# EUROPEAN PATENT SPECIFICATION

(11) **EP 1 618 925 B1**
(45) Date of publication and mention of the grant of the patent: **12.09.2007**
(21) Application number: 04016192.9
(22) Date of filing: 09.07.2004
(51) Int. Cl.: A61Q 1/14, A61K 8/02

(54) **A cosmetic composition for the removal of make-up and an applicator comprising said composition**
Kosmetische Zusammensetzung zur Entfernung von Schminke sowie ein Artikel enthaltend genannte Zusammensetzung
Composition cosmétique pour enlèvement de maquillage et un applicateur comprenant ladite composition

(43) Date of publication of application: 25.01.2006
(73) Proprietor: JOHNSON & JOHNSON GmbH, 40474 Düsseldorf (DE)
(72) Inventor: Lange, Rainer, 53604 Bad Honnef (DE); Rosato, Pietro, 51379 Leverkusen (DE); Hassan, Syed, Burnley, Lancashire BB 102 SD (GB)
(74) Representative: Weber-Bruls, Dorothée

(56) References cited:
- EP-A- 0 422 862
- EP-A- 1 011 630
- EP-A- 1 093 844
- WO-A-01/01949
- WO-A-02/03934
- DE-A- 4 243 272
- DE-A- 19 827 662
- FR-A- 2 829 021
- US-A1- 2002 035 046

## Description

The present invention relates to cosmetic compositions for the removal of make-up, to a method of the preparation of said composition as well as to an applicator comprising said composition.

Numerous compositions for the removal of make-up have already been tested as well as commercialized indicating that until now no solution has been found which satisfies all consumer needs best, especially as far as rather sensitive areas of the skin, e.g. the area around the eye, are concerned. Such compositions shall be non-irritant to the mucous membrane of the eye and not cause any discomfort to the user such as a smarting or stinging sensation.

Whereas on the one hand there is the wish to use rather mild and non-irritant lotions for make-up removal on the other hand nowadays water resistant make-up is becoming more and more popular, however, requiring rather powerful components and applicators for its removal.

In EP 1 011 630 A1 a disposable, single use personal care cleansing and conditioning article for skin care is disclosed which comprises a water insoluble substrate onto which a lathering surfactant has been added. However, it is the construction of this water insoluble substrate which is believed to significantly contribute to the increase in lathering and deposition of conditioning agents and any other active ingredients. This substrate comprises at least two layers one of which is wet extensible and the other one is less wet extensible and which are joined to each other at selected portions.

DE 28 56 716 C2 is about a cosmetic composition for the removal of eye make-up which comprises, besides a preservative, as a surfactant polyethoxylated alpha-methyl glucoside mono- and dialkyl carboxylates the alkyl residue having a chain length of 11 to 21 carbon atoms. The pH value of the composition shall be in the range from 4.5 to 8.5. Optionally this composition also comprises moisturizers, emollients, fragrances and colorants.

Also, DE 38 03 022 A1 provides for a cosmetic eye removal composition which comprises a lathering surfactant, a carrier compound such as a C₁₋₄ alcohol, water or mineral oil, and a mixture of non-ionic, non-lathering, water soluble products of the formula R-O-[C₂H₃₋(CH₂R₁)-O]ₚ-[C₂H₃O-(CH₂OH)]ₙ-H wherein R and R1 are long chain hydrocarbon residues. The latter products help in reducing the aggressive nature of lathering surfactants without spoiling the lathering effect.

The non-lathering cosmetic composition of DE 689 02 302 T2 serves to remove water-proof and water soluble make-up. It comprises an aqueous phase comprising at least one surfactant and an oil phase comprising, for example, vaseline oil, iso-hexadecane, jojoba oil, silicon oil or a synthetic oil. The w/w ratio of the aqueous phase and the oil phase has to be in the range 30/70 to 60/40 and the concentration of the surfactant has to lie in the range from 0.1 to 3 wt. % based on the total weight of the cosmetic composition. With this cosmetic composition it is attempted to not only remove the make-up efficiently but also to provide a good sensation on the skin during and after the application of the composition.

JP 20011278742 A is concerned with a skin cleansing agent having good quality and durability of foam in the massaging removal of a persistent make-up cosmetic yielding also a good feeling in use and exhibiting high cleansing power. This is achieved by using a skin cleansing agent composed of a detergent composition containing a surfactant selected from anionic surfactants, non-ionic surfactants and ampholytic surfactants and a mono-glyceryl ether derivative having a C₄₋₉ alkyl or alkenyl chain.

According to DE 697 00 055 T2 in the oil-in-water emulsions for cosmetic eye make-up removal no surfactant has to be present as it is believed that these compounds are the major cause for irritation of the mucous of the eye. Instead a crosslinked poly(2-acrylamido-2-methylpropane-sulfonic acid) polymer being at least neutralized to an extent of 90 % has to be used.

According to DE 693 10 477 T2 suitable water-in-oil type emulsions shall be obtained by simultaneous use of a silicon surfactant and a fluorinated hydrocarbon.

GB 1 604 951 discloses a non-irritant composition suitable for the removal of eye make-up which comprises in an aqueous solution at least one surface active agent which is an alkyl or hydroxyalkyl polyglycoside in which the alkyl radical has from 11 to 18 carbon atoms and in which the number of glycoside units is from 5 to 25, at least one preservative of a rather specific nature, e.g. sodium ethyl-mercurithiosalicylate, and also a phosphate buffer. With these compositions irritation or smarting sensations in the eye can be avoided allowing also for long-term storage.

According to US 4,543,205 eye make-up can be removed by use of a cosmetic cleansing composition comprising in an aqueous solution a mixture of a glucoside alkyl ether, an amphoteric material having a specific formula, and an active material selected from the group consisting of the monolaurate or the monooleate of sorbitan polyoxyethylinated with 20 mol of ethylene oxide, the monolaurate or the monooleate of glycerol polyoxyethylated with 20 mol of ethylene oxide and a non-ionic polyether compound of a rather specific nature, in aqueous solution. These aqueous systems shall provide excellent make-up removal characteristics, good cosmetic comfort, good innocuousness on ocular testing and excellent long time preservation at widely varying temperatures. However, rather complex mixtures using very specific compounds have to be used to achieve this goal.

In DE 42 43 272 A1 a silicon oil based oil-in-water emulsion is prepared according to the phase inversion technology. Besides a non-ionic emulsifier, e.g. a fatty alcohol polyglycol ether, an addition product of 4 mol ethylene oxide and 1 mol coco fatty alcohol (C_{12/14}-alcohol) and a liquid paraffin oil is used. This PIT emulsion is used in skin and body care products.

DE 198 27 662 A1 discloses technical mixtures of di- and triglycerides, in particular coco glycerides, for use in make-up removal products. These ingredients should allow for a powerful cleansing of waxes, oils, and silicon compounds as well as pigments, and should be rather sensitive to the mucous and to the skin.

Also, in some commercial products two-phase systems are used which have to be shaken vigorously prior to its usage in order to temporarily form a mixture of small aqueous and oily droplets. Unfortunately, when not shaken properly either the oily or the aqueous component can be delivered to an applicator or to the skin to a greater extent yielding a composition which no longer is identical to the original composition. Deviations in the relative amounts of the oily and the aqueous phase usually have a detrimental impact on the cleaning effect.

Against the above, there still exists the need for a very mild, non-irritant cosmetic composition for the removal of make-up of the eye which is yet powerful in removing even water-proof make-up and which provides for a good sensation after the make-up has been removed. It is another object of the present invention to provide a cosmetic composition which, although comprising hydrophilic and hydrophobic ingredients, does not tend to form separated phases on a macroscopic level, even when subjected to long storage times at ambient temperatures.

FR 2 829 021 A1 discloses a make-up removal emulsion containing glycerine, propylene glycol, chondroite sulphate, PEG-32, sodium methyl paraben, polysorbate 21, poloxamer 184, polyglyceryl-3-hydroxylauryl ether, PEG-60 hydrogenated castor oil, cyclopentasiloxane, carbomer, xanthane gum, sodium hydroxide, sodium citrate and water. According to FR 2 829 021 A1 the irritating effects of surfactants in cosmectic compositions can be masked by use of polyholosides.

According to EP 0 422 862 A2 facial cleansing compositions in the form of an oil-in-water emulsion which provide improved cleansing efficacy as well as improved rinsability and improved skin feel can be obtained by use of specific surfactants with an HLB above about 10 in combination with specific polyalphaolefins and specific carboxylic copolymers. These specific surfactants have to be present from about 0.5 to about 10 % and are selected from the group consisting of anionic, non-ionic, zwitterionic, amphoteric and ampholytic surfactants. The specific polyalphaolefins have to be present from about 2 to 30% and have to have the formula R₁-C = C-R₂, wherein R₁ and R₂ are independently from about C₂₀ to about C₃₀ alkyl, wherein said polyalphaolefin has a viscosity of from about 2 to about 4 centistokes at 100°C. Further, the specific carboxylic copolymer has to be present from about 0.025 to about 0.75% and has to comprise polymers of a monomeric mixture containing 95.9 to 98.8 weight percent of an olefinically unsaturated carboxylic monomer selected from the group consisting of acrylic, methacrylic and ethacrylic acids, about 1 to about 3.5 weight percent of an acrylate ester of the formula CH₂=C(R₁)-C(O)-O-R wherein R is an alkyl radical containing 10 to 30 carbon atoms and R, is hydrogen, methyl or ethyl, and 0.1 to 0.6 weight percent of a polymerizable cross-linking polyalkenyl polyether of a polyhydric alcohol containing more than one alkenyl ether group per molecule wherein the parent polyhydric alcohol contains at least 3 carbon atoms and at least 3 hydroxyl groups. With the emulsions of EP 0 422 862 A1 a mineral oil and dimethicone can be simultaneously present.

### Summary of the Invention

The present invention is concerned with a cosmetic composition for the removal of make-up obtained by mixing at least the following ingredients:
a) water,
b) at least one surfactant,
c) at least one emulsion,
d) at least one oil or wax component selected from the group consisting of vegetable, animal and mineral oils and waxes, and
e) at least one silicon oil and/or wax component.

Preferably the cosmetic composition according to the invention has a pH value in the range of 5.0 to 7.5.

In one embodiment the pH value is adjusted by use of a pH adjuster (component f).

In another aspect the cosmetic composition further comprises
g) at least one chelating agent,
h) at least one preservative, and
i) at least one moisturizer.

According to another aspect the present invention provides for the use of micro- or nanoemulsions as the emulsion c). Of course, also micro- and nanoemulsions can be mixed.

Furthermore these emulsions c) can, in addition or alternatively, also comprise an oil-in-water-type emulsion prepared by the phase inversion technique (PIT).

In still another aspect of the present invention it is provided that the emulsion c) comprises, in addition or alternatively, at least one fatty acid mono-, di- and/or triglyceride and/or at least one polyalkoxylated ether of a fatty alcohol as non-ionic emulsifier(s), in particular at least one polyethoxylated C₁₆₋₂₂ alcohol.

In another embodiment it is provided that the emulsion c) comprises, in addition or alternatively, at least one fatty acid ester of a fatty alcohol and/or at least one fatty alcohol as co-emulsifier(s).

Preferred cosmetic compositions are also characterized by an emulsion c) which comprises, in addition or alternatively, at least one ester of a fatty alcohol, in particular cetearyl isononanoate, at least one mono- or diglyceride or a mixture thereof, at least one fatty alcohol, and/or at least one polyethoxylated fatty alcohol.

Emulsion c) of another preferred cosmetic composition comprises at least one oil or wax component selected from the group consisting of vegetable, animal and mineral oils and waxes, in particular a paraffin oil and/or wax component, and/or at least one silicon oil and/or wax component.

According to another embodiment emulsion c) comprises at least one surfactant, at least one moisturizer and/or at least one preservative.

In another aspect of the present invention the cosmetic composition further comprises at least one anti-microbial agent, at least one thickener, at least one fragrance, at least one skin soothing aid, at least one shine control agent, at least one anti-aging agent, at least one anti-acne agent, at least one UV-protection agent, at least one humectant or mixtures thereof.

Further, it is preferred that the silicon oil comprises dimethicone, substituted linear dimethicones, cyclomethicones and mixtures thereof, cyclopentasiloxane being particularly preferred.

According to another preferred embodiment the mineral oil or wax of the cosmetic composition is a paraffin oil or wax, in particular an iso-paraffin oil or wax, more preferably at least one C₅₋₁₈ hydrocarbon, in particular an iso-substituted derivative thereof, iso-hexadecane being particularly preferred.

### Detailed description of the invention

Unless otherwise explicitly specified any percentage whenever used in this description and claims is weight by weight (w/w).

The cosmetic composition according to the invention is preferably liquid or viscous at ambient temperature. More preferably said composition has a viscosity equal or below 300 mPas and in particular equal or below 200 mPas.

As used herein "ambient temperature" refers to a temperature that is in the range of about 20 to about 25 °C.

In general, the cosmetic compositions of this invention are preferably formulated into forms that are used in personal care products that are available in many different forms. These forms include creams, lotions, pastes, liquids, aerosols, soaps, cleansing bars, shampoos and gels any or all of which can be prepared to be also used as a cosmetic product or as a cosmetic and are intended for typical applications to the skin including the scalp and the mucosa including the lips.

In general, the surfactants as used herein may be any of anionic, cationic, non-ionic and amphoteric.

Examples of anionic surfactants comprise fatty acid soaps such as sodium stearate and triethanolamine palmitate, carboxylates, such as alkyl ether carboxylic acids and salts thereof and aminoacid-fatty acid condensates, sulfonates, such as alkane sulfonates, alkene sulfonates, sulfonated fatty acids esters, sulfonated fatty acids amines and alkyl sulfonate formaldehyde condensates, sulfates such as alkyl sulfates, higher secondary alcohol sulfates, alkyl and aryl ether sulfates, fatty acid ether sulfates, fatty acid alkyl olamide sulfates, ether sulfates and turkeky red oil, phosphates, such as alkyl phosphates, ether phosphates, alkyl aryl ether phosphates and amid phosphates, and active agents of N-acylamino acid type.

Examples of cationic surfactants usable herein include amine salts such as alkyl amine salts, polyamines and amino alcohol fatty derivatives, quaternary alkyl ammonium salts, quaternary aryl ammonium salts, pyridinium salts, and imidazolium salts.

Examples of non-ionic surfactants used herein include sorbitan fatty acid esters, glycerine fatty acid esters, polyglycerine fatty acid esters, propylene glycol fatty acid esters, polyethylene glycol fatty acid esters, succrose fatty acid esters, polyoxyethylene alkyl ethers, poly-oxyetholene alkylphenyl ethers, polyoxyethylene fatty acid esters, polyoxyethylene sorbitan fatty acid esters, polyoxyethylene sorbitol fatty acid esters, polyoxyethylene glycerine fatty acid esters, polyoxyethylene glycol fatty acid esters, polyoxyethylene castor oil, polyoxyethylene hydrogenated castor oil, polyoxyethylene phytostanol ether, polyoxyethylene phytosterol ether, polyoxyethylene colestanol ether, polyoxyethylene cholesteryl ether, polyoxyalkylene-modified organopolysiloxane, modified with both polyoxyalkylene and alkyl groups, alkanol amides, sugar ethers and sugar amides.

Examples of amphoteric surfactants usable herein include betaines, aminocarboxylates and imidazoline derivatives.

Suitable surfactants comprise in particular alkyl sulfates, e.g. sodium lauryl sulfates, ammonium lauryl sulfates, sodium cetearyl sulfates; alkyl sulfoacetates, e.g. sodium lauryl sulfoacetates; alkyl ether sulfates, e.g. sodium lauryl sulfates, sodium trideceth sulfates, sodium oleth sulfates, ammonium lauryl sulfates; alkyl ether sulfosuccinates, e.g. disodium lauryl sulfosuccinates; alkyl glycosides, e.g. decyl glycosides; lauryl glucosides; alkyl isocionates; amphoterics, e.g. coco amidopropyl betaine, sodium coco amphoacetates, sodium lauroamphoacetate, disodium lauroamphodiacetate, disodium coco amphodiacetate, sodium lauroamphopropionate, disodium lauroamphodiproprionate, potassium or ammonium salts of the aforementioned amphoterics, capryl/capramidopropyl betain, undecyl amidopropyl betain, lauroamidopropyl betain; and fatty alcohol polyglycol ethers.

The emulsion c) being one constituent of the cosmetic composition of the invention can, for example, be of the oil-in-water or water-in-oil type, or can be of a more complex composition such as water-in-oil-in-water. Oil-in-water type emulsions are particularly preferred.

The aforementioned emulsions preferably represent microemulsions or nanoemulsions.

Preferred cosmetic compositions are those based on emulsions prepared by the so-called phase inversion technique. The phase inversion technique is described in more detail by F. Förster, F. Schambil, and H. Tesmann, Int. J. Cos. Sci. 1990 (12) 217.

According to this technique, oil-in-water formulations made with non-ionic emulsifiers typically undergo a phase inversion upon heating which means that within a particular temperature interval a change of the emulsion type takes place, i.e. from an oil-in-water to a water-in-oil emulsion. The average temperature between that of maximal and of minimal conductivity is referred to as the phase inversion temperature ("PIT").

After heating to a temperature above the PIT, the emulsion is cooled below the PIT whereupon the inverse phase transfer takes place, i.e. from water-in-oil to oil-in-water. The resulting emulsions are usually referred to as "PIT emulsions".

The droplet size of the PIT emulsion depends on a number of factors. PIT emulsions with small droplet size can be obtained with emulsions forming microemulsions having a low surface tension between the oil and water phases at the phase inversion, or that form a laminar liquid crystalline phase.

Preferred are PIT emulsions that are finely dispersed, prefereably in an essentially monodisperse fashion, distributed comprising a small droplet size and having low viscosity.

The oily phase in PIT emulsions may comprise natural oil derivatives, in particular of vegetable origin. Examples are linseed oil, palm oil, olive oil, castor oil, rapeseed oil, soy oil, and in particular peanut oil, coconut oil, sunflower oil and turnip seed oil. The oily phase may further comprise fatty components isolated from these natural oils, i.e. pure triglycerides or mixtures thereof, or the latter components having been prepared chemically. These so-called triglycerides are esters of glycerine with fatty acids or fatty acid mixtures. Preferred triglycerides are those glycerine esters derived from fatty acids, either saturated or unsaturated, having from 10 to 24, particularly from 14 to 20, preferably from 16 to 18 carbon atoms, for example palmitic, heptadecanoic, oleic or stearic acid, or mixtures thereof. Particularly preferred is glyceryl stearate, also referred to as stearin.

The total amount of triglycerides in the PIT formulations may vary. In particular, the total amount of the triglycerides present in the PIT formulations of this invention is in the range of 1-40 %, preferably of 1-30% (w/w), more preferably of 1-20 % (w/w), still more preferably from 1-10 % (w/w) or from 1-5 % (w/w).

The oily phase may further comprise alkyl esters of fatty acids, wherein the alkyl group has from 1 to 30 carbon atoms and the fatty acid has from 8 to 22 carbon atoms. A particular subgroup are the C₁₋₄ alkyl esters of C₁₆₋₁₈ fatty acids, for example of palmitic, heptadecanoic, or stearic acid, in particular the methyl or ethyl esters, including mixtures thereof. A preferred subgroup are the C₈₋₂₂ alkyl esters of C₈₋₂₂ fatty acids. Further preferred subgroups are the C₁₂₋₁₈ alkyl esters, or the C₁₆₋₁₈ alkyl esters, of C₈₋₂₂ fatty acids or of C₁₂₋₁₈ fatty acids or of C₁₆₋₁₈ fatty acids. Fatty acid alkyl esters of particular interest are cetyl, stearyl, cetearyl or palmityl esters; or the esters of the nonanoic, isononanoic, palmic, palmoleic, stearic or isostearic acid; esters composed of the cetyl, stearyl, cetearyl or palmityl esters of the nonanoic, isononanoc, palmic, palmoleic, oleic, stearic or isostearic acid; or mixtures of these esters.

The total amount of alkyl esters of fatty acids in the PIT formulations may vary, and depends on the desired properties of the end formulation. In particular, the total amount of the partial glycerides present in the PIT formulations of this invention is in the range from 1-40 % , preferably from 1-30 % (w/w), more preferably from 1-20 % (w/w), still more preferably from 1-10 % (w/w) or from 1-5 % (w/w).

Of particular interest are oily phases that comprise a vegetable oil or a triglyceride in combination with an alkyl ester of a fatty acid.

The oily phase may further contain fatty acid partial glycerides, in particular C₈₋₂₂ fatty acid partial glycerides. The latter comprise glyceryl mono- or di-fatty acid esters, including mixtures thereof. Preferred are the C₁₂₋₁₈ fatty acid partial glycerides, particularly preferred are the C₁₆₋₁₈ fatty acid partial glycerides, more preferably the C₁₈ fatty acid partial glycerides. Of special interest are glyceryl mono- or distearate, mono- and di-cocoglyceride, and mixtures thereof.

The total amount partial glycerides in the PIT formulations may vary, and depends on the desired properties of the end formulation. In particular, the total amount of the partial glycerides present in the PIT formulations of this invention is in the range from 1-40 %, preferably from 1-30 % (w/w), more preferably from 1-20 % (w/w), still more preferably from 1-10 % (w/w) or from 1-5 % (w/w).

The PIT formulations may further comprise fatty alcohols. Fatty alcohols that can be used are, for example, C₁₂-C₅₀-fatty alcohols, in particular the C₁₂-C₂₄ fatty alcohols, more in particular C₁₂-C₁₈-fatty alcohols that are derived from natural fats, oils or waxes such as, for example, myristyl alcohol, 2-pentadecanol, cetylalcohol, 1-heptadecanol, stearyl alcohol, cetearyl alcohol, 1-nonadecanol, arachidyl alcohol, 1-heneicosanol, behenyl alcohol, brassidyl alcohol, lignoceryl alcohol, cetyl alcohol or myricyl alcohol as well as Guerbet alcohols. Mixtures of fatty alcohols can evidently also be used, including fatty alcohol fractions obtained from the reduction of the corresponding fatty acid fractions derived from naturally occurring oils or fats such as, for example, almond oil, soybean oil, sunflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, and turnip seed oil.

Synthetic alcohols can also be used such as, for example, the linear fatty alcohols of an even number of carbon atoms resulting from the Ziegler-synthesis and also partially branched alcohols resulting from the oxo synthesis.

The use of fatty alcohols advantageously results in the lipid phase having a drier, i.e. less greasy, skin feel, compared to components such as triglycerides.

The total amount of fatty alcohols in the PIT formulations may vary, and depends on the desired properties of the end formulation. In particular, the total amount of the fatty alcohols present in the PIT formulations of this invention is in the range from 1-40 %, preferably from 1-30 % (w/w), more preferably from 1-20 % (w/w), still more preferably from 1-10 % (w/w) or from 1-5 % (w/w).

The PIT formulations may further comprise dialkyl(ene) carbonates. These may be symmetric or asymmetric, straight or branch chained, saturated or unsaturated.

Preferred dialkyl(ene) carbonates are linear or branch chained, saturated or unsaturated C₁₄₋C₃₀-dialkyl(ene) carbonates. More preferred are C₁₆-C₂₂-dialkyl carbonates and amongst these the saturated linear C₁₆-C₂₂-dialkyl carbonates. Particularly preferred is distearyl carbonate. Also liquid dialkyl(ene) carbonates, such as, for example, dihexyl-, dioctyl-, di-(2-ethylhexyl)- or dioleylcarbonate, can be used.

These dialkyl(ene) carbonates can be obtained by re-esterification of dimethyl or diethylcarbonates with the corresponding hydroxyl compounds following art-known procedures. Typical examples of dialkyl(ene) carbonates are re-esterification products of dimethyl- and/or diethylcarbonate with capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaiddyl alcohol, petroseliniyl alcohol, linolyl alcohol, linolenyl alcohol, oleyl alcohol, rizinol alcohol, elaeostearyl alcohol, arachidyl alcohol, gadoleyl alcohol, behenyl alcohol, erucyl alcohol and brassidyl alcohol, Guerbet alcohols, as well as technical mixtures thereof, that can be obtained by hydrogenation of methyl esters derived from suitable oils or fats or oil or fat fractions.

The total amount of dialkyl(ene) carbonates in the PIT formulations may vary, and depend on the desired properties of the end formulation. In particular, the total amount of the dialkyl(ene) carbonates in the PIT formulations may vary, and depends on the desired properties of the end formulation. In particular, the total amount of the dialkyl(ene) carbonates present in the PIT formulations of this invention is in the range of 1-40 % , preferably from 1-30 % (w/w), more preferably from 1-20 % (w/w), still more preferably from 1-10 % (w/w) or from 1-5 % (w/w).

The PIT emulsions may further contain dialkyl(ene) ethers which may be symmetric or asymmetric, straight or branch chained, saturated or unsaturated. Preferred are saturated or unsaturated. Preferred are saturated C₁₆-C₃₀-dalkylethers, in particular C₁₂-C₂₄-dialkylethers or C₁₂-C₁₈-dialkylethers or mixed ethers such as (C₁-C₁₀-alkyl)(C₁₂-C₁₈-alkyl)ethers. More preferred are C₁₆-C₁₈-dialkylethers. Examples are di-n-octylether, di-(2-ethylhexyl)-ether, laurylmethyl, octylbutyl, didodecylether, dicetyl, dicapryl, dilauryl, distearyl, dicetearyl, and dibehenyl ethers.

These ethers can be obtained from the appropriate fatty alcohols in the presence of an acid catalyst following art-known procedures. Typical examples are the products that are obtained by the etherificaion of capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaidyl alcohol, elaeostearyl alcohol, arachidyl alcohol, gadoleyl alcohol, behenyl alcohol, erucyl alcohol and brassidyl alcohol, Guerbet alcohols as well as mixtures thereof, which, for example, are obtained by high pressure hydrogenation of technical mixtures of the methyl esters derived from fats or oils.

The total amount of the dialkyl(ene) ethers in the PIT formulations may vary and depends on the desired properties of the end formulation. In particular, the total amount of the dialkyl(ene) ethers present in the PIT formulations of this invention is in the range from 1-40 %, preferably from 1-30 % (w/w), more preferably from 1-20 % (w/w), still more preferably from 1-10 % (w/w) or from 1-5 % (w/w).

The PIT emulsion further contains a non-ionic emulsifier. Suitable non-ionic emulsifiers comprise:
Polyethoxylated or propoxylated fatty alcohols, fatty acids or C₈₋₁₅ alkylphenols, having 2 to 30 ethoxy units and 0 to 5 propoxy units, or 1 to 5 propoxy units, prepared by reacting the starting alcohols with ethylene or propylene oxide;
Mono- or diesters of polyethoxylated glycerine that with saturated or unsaturated C₁₂-C₁₈ fatty acids, having 1 to 30 ethoxy units;
Glycerine mono- or diesters and sorbitan mono- or diesters of saturated or unsaturated fatty acids as well as ethoxylated derivatives thereof, the latter in particular having from 1 to 30 ethoxy units;
C₈₋₂₂ alkyl mono- or oligoglycosides as well as ethoxylated derivatives thereof, the latter in particular having from 1 to 30 ethoxy units;
ethoxylated castor oil or hydrogenated castor oil, in particular having from 1 to 30 ethoxy units;
polyol fatty acid esters and in particular polyglycerine fatty acid esters, more in particular ricinoleic acid or hydroxyl stearic acid esters; for example polyglycerine poly ricinoleic acid or polyglycerine poly 12-hydroxystearate; and mixtures thereof;
glycerine, polyglycerine, mono- and di-pentaerythrite, sugar derived alcohols such as sorbitol, alkylglucosides and polyglucosides, partially esterified with one or more fatty acids or fatty acid mixtures;
trialkylphosphates as well as polyethoxylated derivatives thereof, the latter in particular having from 1 to 30 ethoxy units;
wool wax alcohols;
polysiloxane-polyalkyl-polyether copolymers and derivatives thereof;
mixed ethers of pentaerythrite, fatty acids, citric acid and fatty alcohols polyalkylene glycols;
glycerine carbonate.

Particular useful emulsifiers for use in the PIT formulations comprise ethoxylated or propoxylated fatty alcohols or fatty acids. Preferred are ethoxylated alcohols, in particular the group of ethoxylated C₈₋₂₂ alcohols; further subgroups of interest among the latter are ethoxylated C₁₂₋₁₈ alcohols or ethoxylated C₁₆₋₁₈ alcohols wherein the number of ethoxyl groups per molecule is in the range of 1 to 35, preferably from 1 to 20, more preferably from 10 to 20.

Examples are ethoxylated cetyl, palmoleyl, stearyl, isostearyl, cetearyl or oleyl alcohol including mixtures thereof.

Accordingly, it is particularly preferred that the emulsion c) comprises at least one polyethoxylated C₁₆₋₂₂ alcohol as a non-ionic emulsifier. In another aspect the emulsion c) comprises preferably at least one ester of a fatty alcohol, at least one mono- or diglyceride or a mixture thereof, at least one fatty alcohol, and at least one polyethoxylated fatty alcohol. It is particularly preferred that as an ester of a fatty alcohol cetearyl isononanoate is used.

Other particular useful emulsifiers comprise an emulsifier system containing a mixture of a hydrophilic and hydrophobic emulsifier.

Hydrophilic emulsifiers comprise ethoxylated or propoxylated fatty alcohols or fatty acids. Preferred are those mentioned above in relation to the particular useful emulsifiers.

Examples of ethoxylated fatty acids are ethoxylated C₁₂₋₂₂ alkylcarbonic acids such as, for example, palmitinic, palmoleinic, stearic, isostearic acid and mixtures thereof, wherein the number of ethoxy groups is in the range of 5 to 50, in particular from 15 to 35.

Hydrophobic emulsifiers comprise polyethoxylated glycerine fatty acid mono- and diesters having 1 to 30 ethoxy units, i.e. polyethoxylated glycerine wherein between 1 and 2 of the hydroxy functions have been esterified with 1 or 2 fatty acids or fatty acid mixtures.

The w/w ratio of the hydrophilic emulsifier components to the hydrophobic emulsifier components is in the range of 10:90 to 90:10, in particular 25:75 to 75:25, more in particular in the range of 40:60 to 60:40.

The total amount of emulsifier in the PIT formulations may vary, and depends on the desired properties of the end formulation. In particular, the total amount of the emulsifier present in the PIT formulations of this invention is in the range from 1-40 %, preferably from 1-30 % (w/w), more preferably from 1-20 % (w/w), still more preferably from 1-10 % (w/w) or from 1-5 % (w/w).

Particularly preferred PIT emulsions for use in the cosmetic and/or dermatological compositions of this invention comprise a mixture of:
(1) an alkyl ester of a fatty acid;
(2) a mono- or diglyceride or a mixture thereof;
(3) a fatty alcohol; and
(4) a polyethoxylated fatty alcohol.

Further particularly preferred PIT emulsions for use in the cosmetic and/or dermatological compositions of this invention comprise a mixture of:
(1) an alkyl ester of a fatty acid;
(2) a mono- or diglyceride or a mixture thereof;
(3) a fatty alcohol;
(4) a polyethoxylated fatty alcohol; and
(5) a dialkyl(ene)ether.

Further particularly preferred PIT emulsions for use in the cosmetic and/or dermatological compositions of this invention comprise a mixture of:
(1) an alkyl ester of a fatty acid;
(2) a mono- or diglyceride or a mixture thereof;
(3) a fatty alcohol; and
(4) a polyethoxylated fatty alcohol;
(6) a dialkyl(ene)carbonate.

Further particularly preferred PIT emulsions for use in the cosmetic and/or dermatological compositions of this invention comprise a mixture of:
(1) an alkyl ester of a fatty acid;
(2) a mono- or diglyceride or a mixture thereof;
(3) a fatty alcohol;
(4) a polyethoxylated fatty alcohol; and
(7) a triglyceride.

More preferred PIT emulsions within said particularly preferred PIT emulsions are those wherein the components (1) - (7) are selected from the particular or preferred groups or subgroups mentioned above.

The concentrations of the components (1) - (7) in the PIT emulsions are as described herein.

The PIT emulsions for use in the products according to the invention in particular contain from 20 to 90 %, more in particular from 30 to 80 % and preferably 30 to 60 % of water. The remainder making up the formulation comprises the oily phase, the emulsifiers and other components. The oily phase typically comprises from 10 to 80 %, in particular from 40 to 70 % of the formulation. The emulsifiers are present in an amount that is in the range of 1 to 25 %, in particular 5 to 20 % and more in particular 5 to 15 %. Preferred are emulsions wherein the w/w ratio of the oil and aqueous phases are about 1:1.

The phase inversion temperature typically is in the range of 40 to 95°C, in particular in the range from 50 to 85°C.

The PIT lotions for use in the present invention can also contain one or more light absorbing or light reflecting substances, in particular those mentioned herein. These can be hydrophilic or hydrophobic. In the former instance these substances will be solved into the aqueous phase while in the latter into the oily phase.

Particular PIT emulsions that can be used in the cosmetic and/or dermatological compositions of this invention are described for example in WO-00/51427 and in WO-00/71676.

It has been found that with decreasing viscosity and/always smaller particle size the compositions become particularly attractive in that they show increased spreading and impregnating properties.

Suitable oils as component d) in particular comprise natural oils or natural oil derivatives, preferably of vegetable origin. Examples are almond oil, soy bean oil, sunflower oil, safflower, corn oil, canola oii, borage oil, evening primrose oil, grapeseed oil, wheat germ oil, avocado oil, jojoba oil, kernel oil, sesame oil, walnut oil, linseed oil, palm oil, olive oil, macadamia oil, castor oil, rapeseed oil, peanut oil, coconut oil, and turnip seed oil and the hardened derivatives thereof. The latter are obtained by hydrogenation of fats or oils. Preferred are hardened oils from vegetable origin, e.g. hardened castor oil, peanut oil, soja oil, turnip seed oil, cotton seed oil, sunflower oil, palm oil, kernel oil, linseed oil, almond oil, olive oil, sesame oil, cocoa butter, shea butter and coconut oil.

The oil component d) alternatively or simultaneously further comprises fatty components isolated from the aforementioned natural oils, i.e. pure triglycerides or mixtures thereof, or the latter components having been prepared chemically. These so-called triglycerides are esters of glycerines with fatty acids or fatty acid mixtures, for example, so-called technical mixtures obtained by hydrolysis from fractions of oils, or by fractioning fatty acid mixtures after hydrolysis. The triglycerides may also be obtained chemically by synthesis.

The cosmetic composition further comprises as a component d), either in addition or alternatively to vegetable, animal and/or mineral oils, at least one wax selected from the group consisting of vegetable, animal and mineral waxes or mixtures thereof. As used herein, the term "wax" refers to oil soluble materials that have a waxy constituency and have a melting point or range of above ambient temperature, in particular above 25°C. Waxes are materials that have a solid to semisolid (creamy) consistency, crystalline or not, being of relative low viscosity a little above their liquefying point. Waxes can be composed of one or more components, synthetic as well as natural, and can in principle be composed of or comprise any oil soluble material having a waxy constituency, including mixtures thereof.

Waxes which can be used may be synthetic or natural waxes, as well as other oil soluble materials that have a waxy consistency.

Natural waxes comprise waxes from vegetable origin, such as purcelline, shea butter, cocoa butter, Japan wax, esparto gras wax, cork wax, Guaruma wax, rice shoot wax, Ouricury wax montan wax, sunflower wax, ceresine wax, sugar cane wax, carnauba wax, candelilla wax, lanolin, fruit-derived waxes, such as orange wax, lemon wax, grapefruit wax and bayberry wax, and the like and of animal origin such as beeswax, woolwax, spermateci and bear fat, shellac wax, and the like. Natural waxes further comprise mineral waxes such as ceresine and ozokerite waxes. Synthetic waxes comprise petroleum-based waxes such as paraffin, vaseline, petrolatum, micro wax. Further synthetic waxes are polyalkylene and polyethylene glycol waxes, e.g. polyethylene wax; waxes based on chlorinated naphtalenes such as "Halowax", synthetic hydrocarbon waxes, and the like, including mixtures thereof. Further waxes are chemically modified waxes, in particular hardened or hydrogenated waxes such as, for example, Montan-ester waxes, Sasol waxes and hydrogenated jojoba waxes.

Preferred among these natural waxes are waxes from vegetable origin.

Other wax components can be certain fats (including mono-, di- and triglycerides and fatty acid alkylesters), fatty alcohols, fatty acids, including substituted fatty acids (in particular hydroxy substituted fatty acids, for example, 12-hydroxystearic acid), dialkyl(ene) ethers, dialkyl(ene) carbonates, dicarboxylic acids (in particular the C₁₆-C₄₀ dialkylesters of dicarboxylic acids, e.g. the C₁₆-C₄₀-dialkylesters of dicarboxylic acids, e.g. the C₁₆-C₄₀- alkyl stearates) and hydroxyl fatty alcohols that comply with the definition of "wax" as outlined herein. Any of these components may contain homologous components that are liquid, as long as the total composition making up the lipid phase has a waxy constituency. For example, waxy fats may contain oils, waxy fatty alcohols may contain liquid fatty alcohols, etc., in such amount that the total composition has a waxy constituency and in particular has the melting point or range specified above.

Still further wax components are selected from the group of aromatic carbonic acids, tricarboxylic acids, or from the group of lactides of long-chained hydroxycarbonic acids.

Further wax components that can be sued are C₃₀-C₅₀-alkyl citrates, e.g. tristearyl citrate, tri-isostearyl citrate, trilauryl citrate; ethylene glycol di-fatty acid esters, in particular the ethylene glycol di-C₁₂-C₃₀-fatty acid esters, e.g. ethylene glycol dipalmitate, ethylene glycol distearate, ethylene glycol di-(12-hydroxystearate).

Examples of hydrocarbon oil which can be used herein include ozokerite, squalane, squalene, ceresine, paraffin, paraffin wax, liquid paraffin.

Particularly preferred are paraffin oil and/or paraffin wax, more preferably at least one iso-paraffin oil and/or iso-paraffin wax. Suitable paraffin oil components comprise at least one C₅₋₁₈ hydrocarbon, in particular an iso-substituted derivative thereof. In particular the paraffin oil comprises iso-hexadecane.

The cosmetic composition according to the invention further comprises as a component e) at least one silicon oil and/or at least one silicon wax component. Such suitable silicons comprise, for example, dimethyl polysiloxane, methylphenylpolysiloxanes, methylhydrogen polysiloxane, the copolymer of dimethyl siloxane and dimethyl phenylsiloxane; cyclic silicons such as octamethyl cyclotetrasiloxanes, decamethyl cyclopentasiloxanes, dodecamethyl cyclohexasiloxane and tetramethyltetrahydrogen cyclotetrasiloxanes; trimethylsiloxysilicate; fluorine-modified silicons; and silicon compounds derivatized with amino-fatty acid, alcohol-, polyether, epoxy-, and/or alkyl groups. Preferred silicon oil components comprise dimethicones, substituted linear dimethicones, cyclomethicones and mixtures thereof. Particularly preferred is cyclopentasiloxane.

According to another aspect of the present invention the cosmetic composition further comprises
g) at least one chelating agent,
h) at least one preservative and/or antioxidant and
i) at least one moisturizer and/or humectant.

Suitable chelating agents, preferably used in small concentrations, comprise, for example, α-hydroxy fatty acid, palmeate acid, phytic acid, lactopherrine, α-hydroxy acids such as citric acid, lactic acid, maleic acid, as well as humic acid, bile acid, bile extracts, bilirubin, biliverdin, EDTH, EGTH, and derivatives thereof; unsaturated fatty acids and derivatives thereof such as for example linolenic acid, linoleic acid, oleic acid; folic acid and derivatives thereof, ubiquinones and ubiquinol and derivatives thereof, vitamin C and derivatives thereof, tocopherol and derivatives thereof (for example vitamin E acetate), Vitamin A and derivatives thereof, coniferyl benzoates of benzoinic acid, rutinic acid and derivatives thereof, α-glycosylrutin, ferula acid, furfurylidene glucital, carnosin, butylhydroxytoluene, hydroxyanisol, nordihydroguaiac resin acid, nor-dihydroguaiaret acid, trihydroxybutyrophenone, ureic acid and derivatives thereof, mannose and derivatives thereof, superoxide-dismutase, zinc and derivatives thereof such as zinc oxide and the like; selenium and derivatives thereof; stilbene and derivatives thereof and derivatives of these active substances that may be used when executing this invention.

In addition, at least one preservative can be added to the formulation of the invention. These preservatives encompass natural and synthetic ingredients of organic or inorganic nature. Suitable preservatives can for example comprise benzoic acid, salts of benzoic acid especially its sodium salt, sorbic acid, especially its potassium salt, formaldehyde, formaldehyde releasing compounds such as DMDM hydantoin, imidazolidinyl urea, diazolidinyl urea alcohols such as ethyl alcohol and/or isopropyl alcohol, glutaraldehyde, parabens, phenoxy ethanols, quaternary ammonium compounds, iodines or iodophors such as iodo-propynyl butylcarbamate (IPBC) and mixtures therof. IPBC in one preferred embodiment is used in combination with at least one polyalkoxylated fatty alcohol, in particular a polyethoxylated fatty alcohol such as PEG-4 laurate and/or PEG-4 dilaurate. Examples of especially suitable preservatives of use in the compositions or formulations of the invention include the C1-C4 alkyl parabens and phenoxyethanol. Preferred preservative mixtures comprise IPBC and DMDM hydantoin, for example marketed as Glydant Plus by Lonza. Most preferably, in particular in terms of effecting very stable cosmetic compositions, IPBC and DMDM hydantoin, or Glydant Plus, respectively, are present in the cosmetic composition in an amount of from about 0.1 to 0.85, preferably in an amount of from about 0.2 to 0.60 wt.-% based on the weight of the cosmetic composition. Furthermore, mixtures comprising chlorphenisin and phenoxy ethanol as for example offered under the trade name Germazide by Collaborative Laboratories are also preferred. Moreover, in addition or as an alternative to parabens such as methyl, ethyl, propyl and butyl parabens or sodium salts of these parabens especially of methyl and propyl parabens, parahydroxy benzoic acids, bronopol, DMDM hydantoin, imidazolidinyl urea, diazolidinyl area and/or benzophenone can be employed. In a particularly preferred embodiment the preservative comprises at least one butylene glycol, IPBC and DMDM hydantoin.

In another preferred embodiment, in particular in terms of effecting very stable cosmetic compositions, the preservative comprises a mixture of IPBC, benzoic acid, dehydroacetic acid and 2-phenoxyethanol, which can for example be prepared by mixing IPBC, phenoxy ethanol and Euxyl^{®} K702, a commercial product of Schülke&Mayr GmbH, Norderstedt. Particularly preferred are also, in particular in terms of effecting very stable cosmetic compositions, preservative mixtures comprising phenoxy ethanol, IPBC and DMDM hydantoin. In both aforementioned mixtures, most preferred, phenoxy ethanol is present in the cosmetic composition in an amount of from about 0.1 to 1.0 wt.-%, preferably in an amount of from about 0.2 to 0.80 wt.-%, and even more preferred in an amount of from about 0.3 to 0.7 wt.-%, based on the weight of the cosmetic composition, IPBC is present in the cosmetic composition in an amount of from about 0.001 to 0.05 wt.-%, preferably in an amount of from about 0.001 to less than 0.02 wt.-%, and even more preferred in an amount of from about 0.05 to 1.9 wt.-%, based on the weight of the cosmetic composition, and DMDM hydantoin or Euxyl^{®} K702, respectively, is present in the cosmetic composition in an amount of from about 0.01 to 0.6 wt.-%, preferably in an amount of from about 0.1 to 0.4 wt.-%, and even more preferred in an amount of from about 0.15 to 0.35 wt.-%, based on the weight of the cosmetic composition.

Preferably, also at least one antioxidant can be present in the cosmetic composition according to the invention. Suitable antioxidants include butylated hydroxyl toluene (BHT), ascorbyl palmitate, butylated hydroanisole (BHA), phenyl-α-naphtylamine, hydroquinone, propyl gallate, nordihydroquiaretic acid, vitamin E or derivatives of vitamin E, vitamin C and derivatives thereof, calcium pantothenic, green tea extracts and mixed polyphenosols, and mixtures thereof of the above. When being utilized the antioxidant can be present in an amount ranging from about 0.02 to about 0.5 % by weight, more preferably from about 0.002 to about 0.1 % by weight of the total composition.

Preferably, polyhydric alcohols can be utilized as humectants in the cosmetic compositions according to the invention. The humectants aid in increasing the effectiveness of the emollient, reduce scaling, stimulate removal of built-up scale and improve skin feel. Suitable polyhydric alcohols include, but are not limited to, glycerol (also known as glycerine), polyalkylene glycols, alkylene polyols and their derivatives, including butylenes glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 5, preferably from about 1 to about 3 percent by weight, based on the total weight of the composition.

According to another aspect, the cosmetic composition according to the present invention further comprises at least one moisturizer. Suitable moisturizers are e.g. butylene glycol, cetyl alcohol, dimethicone, dimyristyl tartrate, glucose, glycereth-26, glycerine, glyceryl stearate, hydrolyzed milk protein, lactic acid, lactose and other sugars, laureth-8, lecithin, octoxyglycering, PEG-12, PEG-135, PEG-150, PEG-20, PEG-8, pentylene glycol, hexylene glycol, phytantriol, polyquaternium-39, PPG-20 methyl glucose ether, propylene glycol, sodium hyaluronate, sodium lactate, sodium PCA, sorbitol, succinoglycan, synthetic beewwax, tri-C14-15 alkyl citrate, and starch or mixtures thereof.

Furthermore, in another aspect of the invention the cosmetic composition further comprises at least one active ingredient, at least one anti-microbial agent, at least one thickener, at least one fragrance, at least one skin soothing aid, at least one shine control agent, at least one anti-aging agent, at least one anti-acne agent, at least one UV-protection agent, or mixtures thereof.

Particularly useful are various active ingredients which are suited to deliver various benefits of the skin or hair during the glancing and/or caring process. In these compositions the product is used for delivering the active ingredient to the skin or hair.

The active ingredients useful herein can be categorized by their therapeutic benefit or their postulated mode of action. However, it is to be understood that the active ingredients useful herein can in some instances provide more than one therapeutic benefit or operate via more than one mode of action. Therefore, classifications herein are made for the sake of convenience and are not intended to limit the active ingredient to that particular application or applications listed. Also, pharmaceutically-acceptable salts of these active ingredients are useful herein. The following active ingredients are useful in the compositions of the present invention.

Examples of useful anti-acne actives include the keratolytics such as salicylic acid (o-hydroxybenzeoic acid), derivatives of salicylic acid such as 5-octanoyl salicylic acid, and resorcinol; retinoids such as retinoic acid and its derivatives (e.g., cis and trans); sulphur-containing D and L amino acids and their derivatives and salts, particularly their N-acetyl derivatives, a preferred example of this is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.

Examples of anti-wrinkle and anti-skin atrophy actives include retinoic acid and its derivatives (e.g., cis and trans); retinol; retinyl esters; salicylic acid and derivatives thereof; sulphur-containing D and L amino acids and their derivatives and salts, particularly the N-acetyl derivatives, a preferred example of which is N-acetyl-L-cysteine; thiols, e.g. ethane thiol; ascorbic acid and derivative thereof; hydroxyl acids, phytic acid, lipoic acid; lysophosphatidic acid, and skin peel agents (e.g., phenol and the like).

Examples of antimicrobial and antifungal actives include β-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorocarbanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomoycin sulfate, chlorhexidine gluconate, chlorhexidine hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycine sulfate, miconazole hydrochloride, amanfadine hydrochloride, amanfadine sulfate, octopirox, parachlorometa xylenol, nystatin, tolnaftate, zinc pyrithione and clotrimazole.

Preferred examples of other actives useful herein include those selected from the group consisting of salicylic acid, benzoyl peroxide, 3-hydroxy benzoic acid, 4-hydroxy benzoic acid, acetyl salicylic acid, 2-hydroxybutanoic acid, 2-hydroxypentanoic acid, 2-hydroxyhexynoic acid, cis-retinoic acid, trans-retinoic acid, retinol, phytic acid, ascorbic acid and derivatives thereof, N-acetyl-L-cysteine, lipoic acid, azelaic acid, arachidonic acid, benzoylperoxider, tetracycline, ibuprofen, naproxen, hydrocortisone, acetaminophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, neocycin sulfate, and mixtures thereof.

Suitable thickeners are e.g. acrylates/steareth-20 methacrylate copolymer, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl silylate, xanthan gum, hydrogenated butylenes/ethylene/styrene copolymer.

Suitable UV protection agents are capable of absorbing the ultraviolet spectrum from sunlight and to emit it as radiation of longer wavelength, in particular infrared radiation, i.e. warms. These agents can be water-soluble as well as oil-soluble. A suitable, non-limiting list of UV protection agents comprising 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof, as well as 4-N,N-(2-ethylhexyl)methylaminobenzoic acid ester of 2,4-dihydroxybenzophenone, 4-N,N-(2-ethylhexyl)methylaminobenzoic acid ester with 4-hydroxydibenzoylmethane, 4-N,N-(2-ethylhexyl)-methylaminobenzoid acid ester of 2-hydroxy-4(2-hydroxyethoxy)benzophenone, 4-N,N-(2-ethylhexyl)-methylaminobenzoic acid ester of 4-(82-hydroxyethoxy)dibenzoylmethane, and mixtures thereof.

Suitable anti-aging agents comprise in particular ethanolamine derivatives preferably an ethanol dialkylamine derivative or a topically acceptable organic salt of zinc and/or magnesium thereof. The organic amine radicals preferably represent independently hydrogen, C₃₋₆ cycloalkyl or C₁₋₆ -alkyl, optionally substituted with hydroxyl, methoxy, oxo or formyl as for example described in WO 03/043596.

Furthermore, notably useful is the incorporation of vitamin A and vitamin A derivatives, including but not restricted to retinol, retinyl palmitate, retinoic acid, retinal, and retinyl propionate. Of particular use are so-called retinoids which encompass retinoic acid or retinoin, retinaldehydes, retinol, their salts, their esters and the stereoisomers thereof. The terms "retinol" and "retinoic acids" should be understood to also include the hydrogenated and non-hydrogenated isomers.

Of course, the above-described additional ingredients and components, e.g. components b), d), e), f), g), h) and/or i) of the cosmetic composition can also be used as individual constituents of the composition and/or can be used in the preparation of the emulsion c), that is, they can be used with the composition without having been added to the emulsion c) in beforehand. Hence, one or more of the aforementioned additional ingredients can be used both with the preparation of the emulsion c) and also as separate ingredients for the preparation of the cosmetic preparation according to the invention.

Preferably, the ingredients a) to e) of the cosmetic composition of the present invention are used in the following amounts:
a) 15 - 93.99 wt. % water,
b) 0.01 - 10 wt. % surfactant,
c) 1 - 25 wt. % emulsion,
d) + e) 5 - 50 wt. % of a mixture of a paraffin oil and/or wax component and a silicon oil and/or wax component.

In particularly preferred cosmetic compositions the w/w ratio of components d) and e) lies in the range from 100:1 to 1:100, preferably in the range from 10:1 to 1:10.

The cosmetic composition according to the present invention is obtained by also admixing at least one oil or wax component selected from the group consisting of vegetable, animal and mineral oils and waxes and of mixtures thereof with the aforementioned components.

Preferably, the cosmetic composition according to the present invention is prepared by mixing the emulsion, in particular the PIT emulsion, (component c)), the oil and/ or wax component selected from the group consisting of vegetable, animal and mineral oils and waxes (component d)), and the silicon oil and/or silicon wax component (component e)), in particular essentially all lipophilic components of the cosmetic composition, separately from other components and/or ingredients. For example, in one preferred embodiment also the perfume and/or the IPBC preservative can be mixed with components c) to e) first. Most suitably, the aforementioned lipophilic components are mixed under vigorous stirring. In particular, mixing is accomplished by homogenizing, which is a well known methodology in the field of the manufacture of emulsions and dispersions, e.g. by use of a so-called rotor/stator-system. For homogenization, for example, Ultratorax devices and/or ultrasonication can be used. In a preferred sequence the oil and/or wax components and/or other lipophilic components are added to the emulsion, in particular to the PIT emulsion, (component c)). In general, it suffices to homogenize the lipophilic premix for an in particular continuous period of about 1 to 30 minutes, preferably from about 2 to 15 minutes.

According to another aspect of the present invention all non-lipophilic components and ingredients of the cosmetic composition, in particular components a) and b) and, if need be, also g), h) and/or i) can be added to the above described premixed lipophilic system. These components or ingredients can be added separately - in any possible sequence or after having been combined - forming an aqueous system. Preferably, these non-lipohilic components and ingredients are added to the above lipophilic premix as a combined aqueous system, either continuously or discontinuously, in particular under stirring. This mixing process can for example be conducted at room temperature. In an alternative embodiment, the above lipophilic premix can also be added to component a), i.e. water, or to a mixture of water and a surfactant (component b)), or even to a mixture of essentially all non-lipophilic components and ingredients, in particular under, preferably vigorous, stirring and/or homogenization.

In a further embodiment it is preferred to adjust the pH of the cosmetic composition after the aqueous system or the non-lipophilic components and ingredients, and the lipophilic premix have been mixed.

When the cosmetic composition is prepared as mentioned above, i.e. in particular by preparing a lipophilic premix separately, especially at least comprising the emulsion, in particular the PIT emulsion, and components d) and e), in particular by vigorous stirring, homogenization and/or ultrasonication, which is then mixed with the remaining components and/or ingredients, in particular at least components a) and b), a very stable cosmetic composition according to the invention is obtained. This composition is stable even when kept at elevated temperatures for long periods of time.

The cosmetic composition according to the present invention is preserved by uses to be very mild and generally non-irritating and non-stinging when applied to the skin, e.g. around the area of the eye. Another advantage of this composition resides in the fact that even on long term storage at ambient temperatures no phase separation on the macroscopic level is observed. As the stability of the cosmetic composition does not deteriorate during usage and/or storage the concentration of individual ingredients does neither increase nor decrease but is kept constant until the last drop. The cosmetic composition can for example be stored at ambient temperatures for more than one year without observing any phase separation, especially when the aforementioned preservative systems are employed and/or when prepared via a premix of components c) to e), i.e. very stable liquid compositions are obtained which can be commercialized as such.

The cosmetic composition according to the invention can be used as such, for example, for the removal of make-up. Preferably, the topical formulations of this invention can be applied on the skin by means of appropriate applicators, such as applicators in the form of sheets such as wipes. Preferred embodiments of the present invention therefore are compositions for use on applicators. Examples of suitable applicators are puffs, pads, wipes, tissues, towels, towelettes, sponges, brushes, cotton balls, gloves, mitts or cotton tip swabs.

The applicators may be made of a variety of materials which are structured such that they are capable of holding and/or absorbing a lotion, in particular the cosmetic composition according to the invention. These materials are therefore preferably porous and/or absorbent in nature.

Advantageously, also when in contact with the applicator material the composition according to the invention does not show any signs of phase separation.

Preferred are applicators which are sheets, more preferably wipes. The sheet of absorbent and/or porous material for use in the products of this invention can take the form of a tissue, a wipe, towel, towelette and the like. This material may also be biodegradable. Suitable sheet materials that can be used can be mono- or multi-layered and/or woven or non-woven, and can be made of one or of several materials. Particularly preferred are non-woven materials that have a web structure of fibrous or filamentous nature in which the fibres or filaments are distributed randomly or with a certain degree of orientation, the former being obtainable by air-laying or by certain wet-laying processes, the latter by other wet-laying or by carding processes. The fibres or filaments can be natural, for example, wood pulp, wool, cotton, linen and the like and/or synthetic, for example, polyvinyls, polyesters, polyolefins, polyamides and the like, or mixtures thereof. Also, man-made natural fibres can be used, such as regenerated cellulose fibres, e.g. rayon, modal or cupro. Among those man-made natural fibres so-called lyocell fibres are particularly preferred.

In one preferred embodiment mixtures of lyocell fibres and synthetic fibres, in particular polyester fibres such as polyethylene terephthalate are used for the non-woven sheet material. This sheet material is preferably made by a process comprising the following steps: carding, cross-lapping and needlepunching.

In general, the sheet material has a weight per square meter in the range of about 10 to about 80 g/m², in particular of 20 to 70 g/m².

Accordingly, a particularly preferred applicator onto which the cosmetic compositions of the present invention can be disposed onto or impregnated into comprises at least one non-woven absorbent sheet comprising 10 to 100 wt. % lyocell fibers and 0 to 90 wt. % of at least one other natural, man-made natural and/or synthetic fiber, wherein the lyocell fiber has a fiber titer in the range from 0.5 to 3.5 dtex, wherein the absorbent sheet has a basis weight in the range from 20 to 500 g/m², and a tensile strength in the range from 5 to 1000 N/5cm in machine direction and in cross direction.

More preferred, the absorbent sheet comprises 30 to 90 wt. %, in particular 40 to 80 wt. %, lyocell fibers and 10 to 70 wt. %, in particular 20 to 60 wt.-%, other natural, man-made natural and/or synthetic fibers, in particular synthetic fibers.

In another preferred embodiment the applicator comprises an absorbent sheet which comprises 10 to 50 wt. %, in particular 10 to 40 wt. %, lyocell fibers, 10 to 40 wt. % natural fibers, preferably fibers made from cotton, and 10 to 80 wt. %, in particular 20 to 80 wt. %, man-made natural and/or synthetic fibers.

In this regard, applicators are preferred comprising an absorbent sheet wherein at least one section of the natural, man-made natural and/or synthetic fiber comprises at least one, in particular permanent, hydrophilic surface.

It is provided that the absorbent sheet comprises 10 to 90 wt. %, in particular 30 to 70 wt. %, natural, 10 to 90 wt. %, in particular 20 to 60 wt. %, man-made natural and/or synthetic fibers which comprise at least a section having at least one, in particular permanent, hydrophilic surface, and 0 to 50 wt. %, in particular 10 to 30 wt. %, polyester fibers.

Preferably, the absorbent sheet has been made by use of carding being the web forming technique and needlepunching being the web bonding technique and wherein the carded fibers have been cross-lapped prior to needlepunching.

With the aforementioned applicator the synthetic fiber comprises preferably a polyester fiber, in particular a PET-fiber.

Particularly favorable results can, for example, be obtained when the non-woven absorbent sheet comprises about 50 wt. % lyocell fibers and about 50 wt. % polyester fibers, in particular PET fibers, especially in terms of softness, strength and bending characteristics.

Lyocell is the name for a generic fiber approved by the Federal Trade Commission in 1996. Lyocell can be characterized as a so called man-made fiber. It is made from wood pulp - and thus has a cellulosic basis - by extrusion of a solution of cellulose in an aqueous tertiary amine N-oxide, for example N-methylmorpholine N-oxide, through a suitable die into an aqueous coagulating bath to produce an assembly of filaments ("solvent spinning"). These filaments are washed with water to remove the solvent and are subsequently dried. According to, for example, US 5,094,690 cellulose is suspended in an aqueous solution of the tertiary amine oxide and is then heated to temperatures between 90 to 120°C with stirring.

Lyocell is more similar to cotton than it is to rayon in many ways. However, like other cellulosic fibers, e. g. viscose, it is breathable, absorbent, and generally comfortable to wear. Lyocell has a high wet strength.

Lyocell fibers can, for example, be purchased from Acordis Cellulosic Fibers under the trade name Tencel or from Lenzing Fibers under the trade name Lenzing lyocell.

In general, natural materials useful with applicators of the present invention encompass silk, keratine and cellulosic fibers. Suitable natural fibers which can be used in combination with lyocell fibers can for example be selected from the group consisting of cotton, wool, silk, linen, sisal, hemp, ramie, flax and jute and mixtures thereof.

Suitable man-made natural fibers can, for example, be selected from the group consisting of regenerated cellulose, in particular viscose, cupro and/or modal, polylactide acid, and alginate, and mixtures thereof. Particularly preferred is regenerated cellulose like viscose.

Non-limiting examples of synthetic fiber materials useful with applicators of the present invention include those selected from the group consisting of acetate fibers, aramide fibers, polyamide fibers, e.g. nylons, polyester fibers, e.g. polyethylene terephthalate fibers (PET), polyolefin fibers, e.g. polypropylene and polyethylene fibers, polyvinyl alcohol fibers, polyurethane fibers or foams, and mixtures thereof. Further suitable synthetic fibers include bi- or tricomponent fibers such as PE/PET- or PP/PE fibers. These fibers can for example be so-called core-sheath-, side-by-side- or island-in-the-sea type fibers.

Preferred embodiments of the applicator according to the invention comprise apart from lyocell fibers also fibers at least a section of which exhibits an, in particular permanent, hydrophilic surface. Such an, in particular permanent, hydrophilic surface can for example be obtained by coating natural, natural man-made or synthetic fibers as described above, e.g. polyamide fibers, e.g. nylons, polyester fibers, e.g. polyethylene terephthalate fibers (PET), polyolefin fibers, e.g. polypropylene and polyethylene fibers, polyvinyl alcohol fibers, bi- or tricomponent fibers such as PE/PET- or PP/PE fibers, viscose fibers, cotton fibers, wool fibers, silk fibers, linen fibers, sisal fibers, hemp fibers, ramie fibers, flax fibers and jute fibers and mixtures thereof, with an, in particular permanent, hydrophilic finish.

A permanent hydrophilic finish can for example be obtained according to WO 96/33303 by applying a first spin finish comprising at least one hydrophilic lubricant which contains polydiorganosiloxane to which after stretching a second spin finish comprising at least one cationic antistatic agent which also comprises polydiorganosiloxane is applied. Also, following the teaching of WO 97/00351 a fiber having a permanent hydrophilic surface finish can be obtained by use of an aqueous dispersion which comprises a hydrophilic copolyester having repeating segments of a polyoxyethylene diester and a polyalkylene diester and a polypropylene oxide polymer capped on one or both ends with an alkyl or ester group wherein said polymer has more than four propylene oxide units and an average molecular weight of at least about 300. In addition, a composition for a permanent hydrophilic finish is disclosed which comprises a mixture of an organic solvent, a hydrophilic copolyester having repeating segments of a polyoxyethylene diester and a polyalkylene diester, and a polypropylene oxide polymer capped on one or both ends with an alkyl or ester group, wherein said polymer has more than four propylene oxide units and an average molecular weight of at least about 300. Furthermore, WO 97/00351 discloses a dispersion comprising a hydrophilic copolyester having repeating segments of a polyoxyethylene diester and a polyalkylene diester, and a polypropylene glycol having an average molecular weight of more than 1100. In addition WO 97/00351 discloses a mixture of an organic solvent, a hydrophilic copolyester having repeating segments of a polyoxyethylene diester and a polyalkylene diester, and a polypropylene glycol having an average molecular weight of more than 1100.

In addition or alternatively synthetic fiber materials are preferred wherein, after their manufacture, hydrophilic compounds being distributed within the fiber material migrate to the surface of these fibers to form a permanent hydrophilic fiber finish. For example, polyester fibers, in particular PET fibers, polypropylene fibers and bicomponent fibers as described above are particularly preferred as the basis or core material for such a permanent hydrophilic surface.

According to WO 98/42898 a permanent hydrophilic fiber surface can be obtained by processing, e.g. extruding, calendering or injection moding, a mixture comprising a polyolefin, a migratable amphiphile such as compounds having polar residues, e.g. carboxyl, hydroxyl, amino, oxazolin, imidazolin, epoxide, isocyanate, ester, ether, amide, alkanol amine and alkanol amide groups, and a transition metal compound such as selected from the group consisting of lead, nickel, zirconium, chromium, titanium and tin salts. Also, from US 6,699,922 B2 non-woven fabrics having a permanent hydrophilic surface can be obtained from synthetic fibers comprised of a polymer containing an effective amount of a di-C₁₀₋₁₂ fatty acid ester of polyethylene glycol.

Suitable polyester fibers having a permanent hydrophilic finish are, for example, commercially available under the trade name Hydrofix^{®} available from DuPont. Suitable polypropylene fibers having a permanent hydrophilic finish are, for example, commercially available under the tradename Hyentangle WA available from Fiber Visions.

In particular, essentially the entire surface of these surface treated natural, natural man-made and synthetic fibers is provided with a permanent hydrophilic finish.

The aforementioned fibers having at least on a section an, in particular permanent, hydrophilic surface can either be blended with lyocell fibers alone or can be blended with lyocell fibers and any of the aforementioned natural, natural man-made or synthetic fibers or any combination thereof to form an absorbent sheet to be used with the applicator according to the present invention. These absorbent products which comprise fibers which exhibit a permanent hydrophilic surface are particularly suited to prevent any liquids disposed onto or impregnated into the absorbent product from migrating downwards through the absorbent product due to gravity. Especially those absorbent products which besides lyocell fibers also comprise fibers which exhibit a permanent hydrophilic surface are preferred. Such a fiber having a permanent hydrophilic surface usually retains its hydrophilic character even upon several exposures to moisture or water washes. For example, the permanent character of a hydrophilic surface or coating can be determent according to EDANA standard no. ERT 150.2-93. Also, in modified EDANA method can be employed, as for example described in WO 96/33303. The modification compared to the standard method resides in the fact that the strike-through measurement is performed three times on precisely the same spot on the absorbent product. The absorbent product is neither dried nor wiped off in any way between the test runs. After three sequences of three test runs each the mean and standard deviations of the measurements are calculated for each test run. In order to be classified as a permanent hydrophilic surface/coating the third strike-through time should be at the most 20 sec., preferably at the most 10 sec., and more preferably at most 5 sec. Alternatively, the permanent character of a hydrophilic surface can also be determined by its re-wetting properties, e.g. measured by EDANA method ERT 151.0-93, i.e. the quantity of fluid (in grams) is measured which flows back into a superposed dry filter paper when a thoroughly wetted fleece is loaded by a 4 kg weight.

Absorbent products being used with the present invention which exhibit a permanent hydrophilic surface are especially suited to retain a liquid or lotion which has been applied to these products at the desired area within this absorbent product. Even when for example treated with a skin care composition and being arranged in a stack of superimposed individual absorbent products the skin care composition will be maintained within its respective absorbent article and will not migrate downwards whereby it can be prevented that those absorbent articles being at the top part of the stack will dry out and the absorbent articles at the bottom part of the stack will be drenched with skin care lotion after a while.

A permanent hydrophilic surface in the sense of the present invention can be defined in such a way that its hydrophilic character does not vanish after repeated exposures to moisture or water washes.

The absorbent product or sheet to be used with the applicator according to the invention can be mono or multi-layered. For example, it can be composed of two, three or more non-woven absorbent sheets as described herein. In addition or alternatively the non-woven absorbent sheet itself can be mono or multi-layered, and can, for example, comprise two, three or more individual sheets.

Particularly preferred are non-woven materials that have a web structure of fibrous or filamentous nature in which fibers or filaments are distributed randomly or with a certain degree of orientation. Non-woven web structures can for example be obtained by airlaying or certain wetlaying processes. At least partially oriented non-woven web structures are accessible via other wetlaying techniques or carding processes. In general, the absorbent sheet can be made by use of web forming techniques selected from the group consisting of carding, spunbonding, meltblowing, airlaying and wetlaying. It is particularly preferred that the non-woven material from which the final absorbent sheet is prepared exhibits at least a certain degree of orientation. Preferably, such an oriented non-woven material is obtained via carding processes.

Preferably, the fibers of the carded non-woven web material are oriented predominantly in a machine direction, and not in cross direction.

Multi-layered sheet materials are herein defined as comprising two or more layers of the same or different non-woven material and/or layers obtained by different techniques, as long as one absorbent sheet complies to the limits of present claim 1. Most preferably, this non-woven absorbent sheet represents the top and/or bottom layer of a multi-layered sheet material.

Multi-layered sheet materials can preferably be made by use of at least one web bonding techniques selected from the group consisting of needlepunching, resin bonding, chemical bonding, thermal bonding, hydroentanglement or a mixture thereof. Most preferred these multi-layered sheet materials are formed by use of hydroentanglement and/or needlepunching, that is adjacently aligned sheets/layers are joined by the aforementioned techniques.

With the hydroentanglement process usually a multitude of very fine water jets are applied to a non-woven substrate carried on a support. Instead of these water jets or simultaneously pressurized air jets can be used.

It is particularly preferred that the non-woven material is cross-lapped prior to the web forming process step. Cross-lapping is well known to a person skilled in the art. For example, the carded web material is moved forward and backwards when laid on a belt or carrier while its lower front portion is pulled perpendicular to this forward and backward movement whereby the web material overlaps in a z-like fashion.

With the use of needlepunching, for example with the aid of one or more needle looms, a web of loose fibers, e.g. a web of carded fibers, is converted into a coherent non-woven fabric. By needlepunching fibers are mechanically oriented through the web. The needles can be arranged on a needle tool, e. g. a needle board or loom, in a non-lined arrangement. In the needle punching step preferably at least one needle tool is used having in the range from about 50 to 300, preferably from about 70 to 250 and more preferably from about 90 to 110 needles per dm². In a preferred embodiment the strokes per minute, the number of needles per loom, the advance rate of the pad and in particular the degree of penetration of the needles is adjusted in such a way that as little as possible energy is imparted on the bed while still obtaining a bonded web system which does not delaminate, preferably without the use of a binder material.

It is has been found that by use of needlepunching a web of high strength can be obtained when using lyocell fibers which still is superior in terms of softness and bulkiness.

It is also preferred with this applicator that the fibers of the absorbent sheet exhibit a fiber titer of below 2.5 dtex, preferably below 2.0 dtex and more preferably equal or below 1.3 dtex. It is particularly preferred when not only the lyocell fiber but also the natural, man-made natural and/or synthetic fibers used with the non-woven absorbent sheet comply to the same general and preferred fiber titer ranges.

In a preferred embodiment the applicator comprises an absorbent sheet which comprises 50 to 65 wt. % lyocell fibers and 35 to 50 wt. % polyester fibers both having a fiber titer of 1.7 dtex, preferably 1.4 dtex, or lower.

In these applicators the absorbent sheet preferably has a thickness in the range from 0.4 to 5 mm, preferably from 1.5 to 3 mm and more preferably from 1.8 to 2.8 mm. Even with a thickness in the range from 1.5 to 3, and more preferably from 1.8 to 2.8 mm the sheet material creates a sensation of softness and bulkiness on the skin. It is therefore possible to achieve such sensations without the need of using excessive amounts of fibers, especially of lyocell.

According to another preferred provision of this applicator the basis weight of the absorbent sheet is in the range from 30 to 400 g/m², preferably in the range from 50 to 250 g/m², and more preferably in the range from 150 to 200 g/m².

In a further aspect the tensile strength of the absorbent sheet is in the range from 20 to 800 N/5cm, preferably from 80 to 400 N/5cm, and more preferably from 100 to 250 N/5cm.

The absorbent sheet exhibits an excellent mechanical strength, in particular the wet strength of this absorbent sheet is very satisfactory. The absorbent product to be used with the applicator according to the invention is therefore resistant to wet collapse and can absorb sufficient amounts of skin care compositions such as emulsions or lotions, or of removed make-up and/or debris without reducing the bulkiness of the sheet material.

According to another embodiment of this applicator the elongation of the absorbent sheet is in the range from 20 to 160 %, preferably in the range from 70 to 120 %, and more preferably in the range from 80 to 100 %.

A further development of this applicator provides for the bending torque of the absorbent product, in particular of the absorbent sheet, to be equal or below 0.20, preferably equal or below 0.17, and more preferably equal or below 0.15 g × cm, according to the Kawabata test in particular both in machine direction and in cross direction.

The absorbent products to be used with the applicator of the present invention are superior in terms of softness and strength. In particular, the soft sensation of the absorbent material as such as well as the ease with which it can be bent contribute to a favorable impression when used on the skin either in dry form or after having been impregnated with a lotion. Absorbent products, in particular absorbent sheets being used with absorbent products, are most suited when having a bending torque of no more than 0.2 g × cm, preferably of no more than 0.17 g × cm, in particular of no more than 0.15 g × cm. It has been found that those absorbent products are especially superior in terms of softness which comply with the aforementioned bending torque ranges in the machine direction as well as in cross direction.

The bending torque in the meaning of the present invention is measured by use of a Kawabata KES-FB2 pure bending tester. The Kawabata bending test is part of the Kawabata system which is designed to measure basic mechanical properties of non-wovens and other web materials. With this test the bending torque is established by gathering the results of at least three samples and calculating the average from these test results. The sample's size is 8.9 cm × 8.9 cm. Further, the calibration mass is 50 g, and the instrument sensitivity is equal to 5 × 1. The front moving jaw to rear moving jaw gap is set to 1 cm. There is no side orientation of the samples. There are four bending cycles per measurement. The cycle curvature is equal to 0 cm⁻¹ to ^{+2,5} cm⁻¹ to -2,5 cm⁻¹ to 0 cm⁻¹. The cycle rate is 0.5 cm⁻¹/sec, and the number of measurements is set to 8. The bending torque (gxcm) then represents the slope of the linear regression line between approximately 0.5 cm⁻¹ and 1.5 cm⁻¹ of the Moment (g × cm/cm) versus Curvature (1/cm curve).

In a further aspect the tensile strength of the absorbent sheet is in the range from 20 to 800 N/5cm, preferably from 80 to 400 N/5cm, and more preferably from 100 to 250 N/5cm.

It is also preferred that least one absorbent sheet of this applicator comprises at least one three-dimensional surface pattern, in particular at least one embossment.

In a preferred embodiment of the aforementioned applicator the absorbent sheet has a density of below 0,09 g/cm³.

A three-dimensional surface pattern of the absorbent product can for example be obtained by the needle punching technique. Due to a variation in, for example, needle density per area, needling depths and intensity and/or needle size and/or shape a pattern of raised and lowered regions can be obtained. Furthermore, such a structuring or patterning of the surface of the absorbent product can also be obtained by use of calendar roles or other known conventional embossment techniques.

Interestingly, although the absorbent products used with the applicators according to the invention exhibit a lower basis weight than the comparative sheet materials a higher tensile strength in machine direction as well as in cross direction can be obtained. Furthermore, although less material is needed thicker pads can for example be obtained. In addition, even with very thin sheets a sensation of softness and bulkiness is achieved which cannot be obtained with conventional pad materials of similar thickness. Also, the absorbent products used with the applicators according to the invention are superior with respect to their absorption characteristics. Thus, it is also a major benefit of the absorbent product as used with the applicator of the present invention that it easily absorbs high quantities of fluids or lotions, but that it is also capable of releasing sufficient amounts of skin care and/or cleansing compositions which have been impregnated into these products, in particular when also fibers having, in particular a permanent, hydrophilic surface are employed. Absorbency and retaining characteristics can for example be determined by use of the EDANA 10.3-99 test. With this test usually a 10 cm × 10 cm fiber specimen is deposited in a liquid bath until saturated. Then, the specimen is held in a vertical orientation to let the liquid drip off for 2 minutes, after which the weight of the soaked specimen as well as the amount of absorbed liquid is determined.

The aforementioned specific embodiments of an applicator which comprises an amount of lyocell fibers can be incorporated into pads, towels, towelettes, tissues, puffs, wipes, sponges, brushes, cotton balls, gloves, mitts or cotton tip swabs, pads being particularly preferred. The aforementioned applicators can according to another aspect of the present invention be used in the form of a product assembly which comprises two or three or more applicators as described above which have been treated with the cosmetic composition of the present invention.

As used herein with respect to non-woven webs the term "machine direction" refers to the direction of web travel as the non-woven web is produced, for example, on commercial non-woven manufacturing equipment. Likewise, the term "cross direction" refers to the direction of the plane of the web perpendicular to the machine direction.

As used herein, the term "basis weight" means the weight per unit area of the absorbent product, or the non-woven absorbent sheet. The units of basis weight are typically expressed in grams per square meter.

The invention is further illustrated by the following examples.

### Example 1: Formulation A

| | |
|---|---|
| Water | 73.395 % |
| Tetrasodium EDTA | 0.08 % |
| Coco-glucoside | 0.275 % |
| Phenoxyethanol | 0.9 % |
| Nipastat¹⁾ | 0.3 % |
| PIT emulsion²⁾ | 10 % |
| Cyclopentasiloxane | 10% |
| Isohexadecane | 3 % |
| Glycerine | 2 % |
| Citric acid | 0.05 % |

| | |
|---|---|
| ¹⁾ Nipastat is a preservative comprising butyl paraben, ethyl paraben, isobutyl paraben, methyl paraben and propyl paraben; ²⁾ The PIT emulsion comprises water, ceteareth-12, ceteareth-20, cetearyl alcohol, cetearyl isononanoate, cetyl palmitate, glycerine and glycerol stearate, also commercially available under the trade name Emulgade CM; Ceteareth-12 is ethoxylated cetostearyl (or cetearyl) alcohol having 12 ethoxy units. Ceteareth-20 is ethoxylated cetostearyl alcohol having 20 ethoxy units; | |

### Example 2: Formulation B

| | |
|---|---|
| Water | 73.395 % |
| Tetrasodium EDTA | 0.08 % |
| Coco-glucoside | 0.275 % |
| Phenoxyethanol | 0.9 % |
| Glydant Plus Liquid³⁾ | 0.3 % |
| PIT emulsion²⁾ | 10 % |
| Cyclopentasiloxane | 10% |
| Isohexadecane | 3 % |
| Glycerine | 2 % |
| Citric acid | 0.05 % |

| | |
|---|---|
| ²⁾ The PIT emulsion comprises water, ceteareth-12, ceteareth-20, cetearyl alcohol, cetearyl isononanoate, cetyl palmitate, glycerine and glycerol stearate, also commercially available under the trade name Emulgade CM; Ceteareth-12 is ethoxylated cetostearyl (or cetearyl) alcohol having 12 ethoxy units. Ceteareth-20 is ethoxylated cetostearyl alcohol having 20 ethoxy units; ³⁾ Glydant Plus Liquid is a preservative comprising water, butylene glycol, iodopropynyl butylcarbamate (IPBC), and DMDM hydantoin; | |

### Example 3: Formulation C

| | |
|---|---|
| Water | 73.395 % |
| Tetrasodium EDTA | 0.08 % |
| Coco-glucoside | 0.275 % |
| Phenoxyethanol | 0.4 % |
| Glycasil L⁴⁾ | 0.8 % |
| Euxyl K 702⁵⁾ | 0.3 % |
| PIT emulsion²⁾ | 10 % |
| Cyclopentasiloxane | 10% |
| Isohexadecane | 3 % |
| Glycerine | 2 % |
| Citric acid | 0.05 % |

| | |
|---|---|
| ²⁾ The PIT emulsion comprises water, ceteareth-12, ceteareth-20, cetearyl alcohol, cetearyl isononanoate, cetyl palmitate, glycerine and glycerol stearate, also commercially available under the trade name Emulgade CM; Ceteareth-12 is ethoxylated cetostearyl (or cetearyl) alcohol having 12 ethoxy units. Ceteareth-20 is ethoxylated cetostearyl alcohol having 20 ethoxy units; ⁴⁾ Glycasil L is a preservative comprising IPBC, PEG-4 laurate and PEG-4 dilaurate; and ⁵⁾ Euxyl K 702 is a preservative comprising water, benzoic acid, dehydroacetic acid and 2-phenoxyethanol. | |

The above ingredients are mixed under stirring and subsequently either directly applied to the skin for the removal of make-up or applied to an applicator, such as a wipe.

In particular, the formulations B and C are very mild and non-stinging when applied to the skin, even when applied to the skin area around the eyes.

Obviously, numerous modifications and variations of the present invention are possible in light of the above teachings. It is therefore to be understood that within the scope of the appended claims, the invention may be practiced otherwise than as specifically described herein.

## Claims

1. A cosmetic composition for the removal of make-up obtained by mixing at least the following ingredients:
a) water,
b) at least one surfactant,
c) at least one emulsion,
d) at least one oil or wax component selected from the group consisting of vegetable, animal and mineral oils and waxes, and
e) at least one silicon oil and/or wax component.

2. The cosmetic composition according to claim 1 further comprising at least one pH adjuster (f).

3. The cosmetic composition according to claim 1 or 2 further comprising
g) at least one chelating agent,
h) at least one preservative, and/or
i) at least one moisturizer.

4. The cosmetic composition according to claim 3 wherein the preservative comprises a mixture comprising iodo-propynyl butyl-carbamate (IPBC) and DMDM hydantoin, in particular in an amount of from about 0.1 to 0.85wt%, based on the weight of the cosmetic composition; a mixture comprising IPBC, benzoic acid, dehydroacetic acid and phenoxyethanol; and/or a mixture comprising phenoxy ethanol, in particular in an amount of from about 0.1 to 1.0 wt.-%, based on the weight of the cosmetic composition, IPBC, in particular in an amount of from about 0.001 to 0.05 wt.-% based on the weight of the cosmetic composition, and DMDM hydantoin, in particular in an amount of from about 0.01 to 0.6 wt.-%, based on the weight of the cosmetic composition.

5. The cosmetic composition according to one of the preceding claims wherein the emulsion c) comprises a microemulsion, a nanoemulsion, or an oil-in-water type emulsion prepared by the phase inversion technique (PIT), each comprising at least one oil phase.

6. The cosmetic composition according to one of the preceding claims wherein the emulsion c) comprises at least one fatty acid mono-, di- and/or tri-glyceride and/or at least one polyalkoxylated ether of a fatty alcohol as non-ionic emulsifier(s), in particular a polyethoxylated C₁₆₋₂₂ alcohol.

7. The cosmetic composition according to one of the preceding claims wherein the emulsion c) comprises at least one fatty acid ester of a fatty alcohol and/or at least one fatty alcohol, or wherein the emulsion c) comprises at least one ester of a fatty alcohol, at least one mono- or diglyceride or a mixture thereof, or wherein the emulsion c) comprises at least one fatty alcohol, and at least one polyethoxylated fatty alcohol.

8. The cosmetic composition according to one of the preceding claims wherein the oil phase of the emulsion c) comprises at least one ester of a fatty alcohol, in particular cetearyl isononanoate.

9. The cosmetic composition according to one of the preceding claims wherein the emulsion c) further comprises at least one dialkyl(ene) ether.

10. The cosmetic composition according to one of the preceding claims wherein the emulsion c) comprises at least one oil or wax component selected from the group consisting of vegetable, animal and mineral oils and waxes, in particular a paraffin oil and/or wax component, and/or at least one silicon oil and/or wax component.

11. The cosmetic composition according to one of the preceding claims wherein the emulsion c) further comprises at least one surfactant, at least one moisturizer and/or at least one preservative.

12. The cosmetic composition according to one of the preceding claims further comprising
at least one anti-microbial agent, at least one thickener, at least one fragrance, at least one skin soothing aid, at least one shine control agent, at least one anti-aging agent, at least one anti-acne agent, at least one UV-protection agent, at least one humectant or mixtures thereof.

13. The cosmetic composition according to one of the preceding claims wherein the silicon oil comprises dimethicone, substituted linear dimethicones, cyclomethicones and mixtures thereof, in particular cyclopentasiloxane.

14. The cosmetic composition according to one of the preceding claims wherein the mineral oil or wax is a paraffin oil or wax, in particular an iso-paraffin oil or wax.

15. The cosmetic composition according to one of the preceding claims wherein the paraffin oil component comprises at least one, in particular iso-substituted, C₅₋₁₈ hydrocarbon, in particular iso-hexadecane.

16. The cosmetic composition according to one of the preceding claims wherein the ingredients are mixed in the following amounts:
a) 15 - 93.99 wt. % water,
b) 0.01 - 10 wt. % surfactant,
c) 1 - 25 wt. % emulsion,
d) + e) 5 - 50 wt. % of a mixture of a paraffin oil and/or wax component and a silicon oil and/or wax component.

17. The cosmetic composition according to one of the preceding claims wherein the w/w ratio of components d) and e) lies in the range from 100: 1 to 1:100, preferably in the range from 10:1 to 1:10.

18. An applicator for cosmetic and/or dermatological skin caring and/or cleansing purposes comprising at least one cosmetic composition according to one of the preceding claims.

19. The applicator according to claim 18 comprising at least one non-woven absorbent sheet comprising 10 to 100 wt. % lyocell fibers and 0 to 90 wt. % of at least one other natural, man-made natural and/or synthetic fiber, in particular a polyester fiber, wherein the lyocell fiber has a fiber titer in the range from 0.5 to 3.5 dtex, wherein the absorbent sheet has a basis weight in the range from 20 to 500 g/m², and a tensile strength in the range from 5 to 1000 N/5cm in machine direction and in cross direction.

20. The applicator according to claim 18 or 19 wherein the absorbent sheet comprises 30 to 90 wt. %, in particular 40 to 80 wt. %, lyocell fibers and 10 to 70 wt. %, in particular 20 to 60 wt.-%, other natural, man-made natural and/or synthetic fibers, in particular synthetic fibers.

21. The applicator according to one of claims 18 to 20 wherein the absorbent sheet comprises 10 to 50 wt. %, in particular 10 to 40 wt. %, lyocell fibers, 10 to 40 wt. % natural fibers, preferably fibers made from cotton, and 10 to 80 wt. %, in particular 20 to 80 wt. %, man-made natural and/or synthetic fibers, in particular a polyester fiber, wherein at least one section of the natural, man-made natural and/or synthetic fiber in particular comprises at least one, in particular permanent, hydrophilic surface.

22. The applicator according to one of claims 18 to 21 the absorbent sheet has been made by use of carding being the web forming technique and needlepunching being the web bonding technique and wherein the carded fibers have been cross-lapped prior to needlepunching.

23. The applicator according to one of claims 18 to 22 wherein the fibers of the absorbent sheet exhibit a fiber titer of below 2.5 dtex, preferably below 2.0 dtex and more preferably equal or below 1.3 dtex.

24. The applicator according to one of claims 18 to 23 wherein the absorbent sheet has a thickness in the range from 0.4 to 5 mm, preferably from 1.5 to 3 mm and more preferably from 1.8 to 2.8 mm.

25. The applicator according to one of claims 18 to 24 wherein the bending torque of the absorbent product, in particular of the absorbent sheet, is equal or below 0.20, preferably equal or below 0.17, and more preferably equal or below 0.15 g × cm, according to the Kawabata test, in particular both in machine direction and in cross direction.

26. An applicator assembly comprising two, three or more applicators according to one of claims 18 to 25 being at least partially directly or indirectly imposed onto each other, in particular arranged in at least one stack or on a role.

27. A method for the preparation of the cosmetic composition according to one of claims 1 to 18 comprising the steps of
a1) mixing at least one emulsion, in particular PIT emulsion, (component c)), at least one oil or wax component selected from the group consisting of vegetable, animal and mineral oils and waxes (component e)), and at least one silicon oil and/or silicon wax component (component f)) to form a lipophilic premix, in particular by homogenization,
b1) mixing water (component a)) and at least one surfactant (component b)) to form an aqueous system, and
c1) mixing the lipophilic premix obtained under a) and the aqueous system obtained under b) to form a stable cosmetic composition, in particular by adding the aqueous system to the lipophilic premix; or
a2) mixing at least one emulsion, in particular PIT emulsion, (component c)), at least one oil or wax component selected from the group consisting of vegetable, animal and mineral oils and waxes (component e)), and at least one silicon oil and/or silicon wax component (component f)) to form a lipophilic premix, in particular by homogenization, and
b2) mixing the lipophilic premix obtained under a) with water (component a)) and at least one surfactant (component b)) to form a stable cosmetic composition, in particular by adding water and surfactant to the lipophilic premix.

28. The method according to claim 27 further comprising the step of adding at least one chelating agent (component g)), at least one moisturizer (component i)) and/or at least one further hydrophilic ingredient in step b1) or b2), and/or adding at least one preservative (component h)), perfume and/or at least one further lipophilic ingredient in step a1) or a2).

29. The method according to claim 27 or 28 further comprising the step of adjusting the pH of the cosmetic composition obtained in step c1) or b2) to a range from 5,5 to 7,5 by adding a pH adjuster (f).

30. The method according to one of claims 27 to 29 wherein at least the mixing in step a1) or a2) is accomplished by use of homogenization.

## Patentansprüche

1. Kosmetische Zusammensetzung zur Entfernung von Make-up, erhalten durch das Vermischen von zumindest folgenden Bestandteilen:
a) Wasser
b) zumindest ein grenzflächenaktiver Stoff
c) zumindest eine Emulsion
d) zumindest eine Öl- oder Wachskomponente, ausgewählt aus der Gruppe, bestehend aus pflanzlichen, tierischen und mineralischen Ölen und Wachsen, und
e) zumindest einer Silikonöl- und/oder Silikonwachskomponente

2. Kosmetische Zusammensetzung nach Anspruch 1, ferner umfassend zumindest einen pH-Regler (f).

3. Kosmetische Zusammensetzung nach Anspruch 1 oder 2, ferner umfassend
g) zumindest ein chelatisierendes Mittel
h) zumindest ein Konservierungsmittel, und/oder
i) zumindest ein Feuchtemittel.

4. Kosmetische Zusammensetzung nach Anspruch 3, **dadurch gekennzeichnet, dass**
das Konservierungsmittel ein Gemisch umfasst, welches Jodpropynylbutylcarbamat (IPBC) und DMDM Hydantoin umfasst, im besonderen in einer Menge von zirka 0,1 bis 0,85 Gewichtsprozent, auf der Basis des Gewichts der kosmetischen Zusammensetzung; ein Gemisch, umfassend IPBC, Benzoesäure, Dehydroessigsäure und Phenoxyethanol; und/oder ein Gemisch, umfassend Phenoxyethanol, im besonderen in einer Menge von zirka 0,1 bis 1,0 Gewichtsprozent, auf der Basis des Gewichts der kosmetischen Zusammensetzung, IPBC, im besonderen in einer Menge von zirka 0,001 bis 0,05 Gewichtsprozent, auf der Basis des Gewichts der kosmetischen Zusammensetzung, und DMDM Hydantoin, im besonderen in einer Menge von zirka 0,01 bis 0,6 Gewichtsprozent, auf der Basis des Gewichts der kosmetischen Zusammensetzung.

5. Kosmetische Zusammensetzung nach einem der vorangehenden Ansprüche, **dadurch gekennzeichnet, dass** die Emulsion c) eine Mikroemulsion, eine Nanoemulsion oder eine Emulsion des Öl-in-Wasser-Typs, zubereitet nach der Phasenumkehrtechnik (PIT), umfasst, wobei jede zumindest eine Ölphase umfasst.

6. Kosmetische Zusammensetzung nach einem der vorangehenden Ansprüche, **dadurch gekennzeichnet, dass** die Emulsion c) zumindest ein Fettsäure-mono-, -di- und/oder -triglycerid und/oder zumindest einen polyalkoxylierten Ether eines Fettalkohols als nichtionogenen Emulgator/nichtionogene Emulgatoren, im besonderen einen polyethoxylierten C₁₆₋₂₂ Alkohol, umfasst.

7. Kosmetische Zusammensetzung nach einem der vorangehenden Ansprüche, **dadurch gekennzeichnet, dass** die Emulsion c) zumindest einen Fettsäureester eines Fettalkohols und/oder zumindest einen Fettalkohol umfasst, oder wobei die Emulsion c) zumindest einen Ester eines Fettalkohols, zumindest ein mono- oder Diglycerid oder ein Gemisch derselben umfasst oder wobei die Emulsion c) zumindest einen Fettalkohol und zumindest einen polyethoxylierten Fettalkohol umfasst.

8. Kosmetische Zusammensetzung nach einem der vorangehenden Ansprüche, **dadurch gekennzeichnet, dass** die Ölphase der Emulsion c) zumindest einen Ester eines Fettalkohols, im besonderen Cetearylisononanoat umfasst.

9. Kosmetische Zusammensetzung nach einem der vorangehenden Ansprüche, **dadurch gekennzeichnet, dass** die Emulsion c) ferner zumindest einen Dialkyl(en)-Ether umfasst.

10. Kosmetische Zusammensetzung nach einem der vorangehenden Ansprüche, **dadurch gekennzeichnet, dass** die Emulsion c) zumindest eine Öl- oder Wachskomponente umfasst, ausgewählt aus der Gruppe, bestehend aus pflanzlichen, tierischen und mineralischen Ölen und Wachsen, im besonderen eine Paraffinöl- und/oder -wachskomponente, und/oder zumindest eine Silikonöl- und/oder -wachskomponente.

11. Kosmetische Zusammensetzung nach einem der vorangehenden Ansprüche, **dadurch gekennzeichnet, dass** die Emulsion c) weiterhin zumindest einen grenzflächenaktiven Stoff, zumindest ein Feuchtemittel und/oder zumindest ein Konservierungsmittel umfasst.

12. Kosmetische Zusammensetzung nach einem der vorangehenden Ansprüche, ferner umfassend zumindest einen antimikrobiellen Wirkstoff, zumindest ein Verdickungsmittel, zumindest einen Duftstoff, zumindest ein Mittel zum Geschmeidigmachen der Haut, zumindest ein Mittel zur Kontrolle des Glanzes, zumindest ein Anti-Aging-Mittel, zumindest ein Anti-Akne-Mittel, zumindest ein UV-Schutzmittel, zumindest ein Feuchthaltemittel oder Gemische derselben.

13. Kosmetische Zusammensetzung nach einem der vorangehenden Ansprüche, **dadurch gekennzeichnet, dass** das Silikonöl Dimethicon, substituierte lineare Dimethicone, Cyclomethicone und Gemische derselben, im besonderen Cyclopentasiloxan, umfasst.

14. Kosmetische Zusammensetzung nach einem der vorangehenden Ansprüche, **dadurch gekennzeichnet, dass** das Mineralöl oder -wachs ein Paraffinöl oder -wachs ist, im besonderen ein Isoparaffinöl oder -wachs.

15. Kosmetische Zusammensetzung nach einem der vorangehenden Ansprüche, **dadurch gekennzeichnet, dass** die Paraffinölkomponente zumindest einen, im besonderen iso-substituierten, C₅₋₁₈ Kohlenwasserstoff, im besonderen iso-Hexadecan, umfasst.

16. Kosmetische Zusammensetzung nach einem der vorangehenden Ansprüche, **dadurch gekennzeichnet, dass** die Bestandteile in den folgenden Mengen gemischt sind:
a) 15 - 93,99 Gewichtsprozent Wasser
b) 0,01- 10 Gewichtsprozent grenzflächenaktiver Stoff
c) 1-25 Gewichtsprozent Emulsion
d)+e) 5-50 Gewichtsprozent Gemisch aus einer Paraffinöl- und/oder -wachskomponente und einer Silikonöl- und/oder -wachskomponente.

17. Kosmetische Zusammensetzung nach einem der vorangehenden Ansprüche, **dadurch gekennzeichnet, dass** das w/w-Verhältnis der Komponenten d) und e) im Bereich von 100:1 bis 1:100, vorzugsweise im Bereich von 10:1 bis 1:10 liegt.

18. Applikator für kosmetische und/oder dermatologische Hautpflege-und/oder Hautreinigungszwecke, umfassend zumindest eine kosmetische Zusammensetzung nach einem der vorangehenden Ansprüche.

19. Applikator nach Anspruch 18, umfassend zumindest eine absorbierende Vliesstofflage, welche 10 bis 100 Gewichtsprozent Lyocell-Fasern und 0 bis 90 Gewichtsprozent von zumindest einer anderen Naturfaser, einer künstlich hergestellten Naturfaser und/oder synthetischer Faser, im besonderen einer Polyesterfaser, wobei die Lyocell-Faser einen Fasertiter im Bereich von 0,5 bis 3,5 dtex, die absorbierende Lage ein Basisgewicht im Bereich von 20 bis 500 g/m² und eine Zugfestigkeit im Bereich von 5 bis 1000 N/5cm in Maschinenrichtung und in Querrichtung aufweisen.

20. Applikator nach Anspruch 18 oder 19, **dadurch gekennzeichnet, dass** die absorbierende Lage 30 bis 90 Gewichtsprozent, im besonderen 40 bis 80 Gewichtsprozent, Lyocell-Fasern und 10 bis 70 Gewichtsprozent, im besonderen 20 bis 60 Gewichtsprozent, von anderen Naturfasern, künstlich hergestellten Naturfasern und/oder synthetischen Fasern, im besonderen synthetischen Fasern, umfasst.

21. Applikator nach einem der Ansprüche 18 bis 20, **dadurch gekennzeichnet, dass** die absorbierende Lage 10 bis 50 Gewichtsprozent, im besonderen 10 bis 40 Gewichtsprozent, Lyocell-Fasern, 10 bis 40 Gewichtsprozent Naturfasern, vorzugsweise aus Baumwolle hergestellte Fasern, und 10 bis 80 Gewichtsprozent, im besonderen 20 bis 80 Gewichtsprozent, künstlich hergestellte Natur- und/oder synthetische Fasern, im besonderen eine Polyesterfaser, umfasst, wobei zumindest ein Teil der Naturfasern, der künstlich hergestellten Natur- und/oder synthetischen Fasern im besonderen zumindest eine, insbesondere permanente, hydrophile Oberfläche umfasst.

22. Applikator nach einem der Ansprüche 18 bis 21, **dadurch gekennzeichnet, dass** die absorbierende Lage durch Einsatz des Kardierens als Gewebeausbildungsverfahren und des Nadelns als Gewebebondingverfahren hergestellt wurde, und wobei die kardierten Fasern vor dem Nadeln kreuzweise überlappt wurden.

23. Applikator nach einem der Ansprüche 18 bis 22, **dadurch gekennzeichnet, dass** die Fasern der absorbierenden Lage einen Fasertiter unterhalb von 2,5 dtex, vorzugsweise unterhalb von 2,0 dtex und noch besser von gleich oder unterhalb 1,3 dtex aufweisen.

24. Applikator nach einem der Ansprüche 18 bis 23, **dadurch gekennzeichnet, daß** die absorbierende Lage eine Dicke im Bereich von 0,4 bis 5 mm, vorzugsweise von 1,5 bis 3 mm und noch besser von 1,8 bis 2,8 mm, aufweist.

25. Applikator nach einem der Ansprüche 18 bis 24, **dadurch gekennzeichnet, dass** die Biege-Verdrehungskraft des absorbierenden Produkts, im besonderen der absorbierenden Lage, gleich oder unterhalb von 0,20, vorzugsweise gleich oder unterhalb von 0,17 und besser noch gleich oder unterhalb von 0,15 g × cm nach dem Kawabata Test ist, insbesondere sowohl in Maschinenrichtung wie auch in Querrichtung.

26. Applikatoranordnung, umfassend zwei, drei oder mehr Applikatoren nach einem der Ansprüche 18 bis 25, die zumindest zum Teil direkt oder indirekt übereinander gelegt sind, im besonderen in zumindest einem Stapel oder auf einer Rolle angeordnet.

27. Verfahren für die Herstellung der kosmetischen Zusammensetzung nach einem der Ansprüche 1 bis 18, umfassend folgende Schritte:
a1) Vermischen von zumindest einer Emulsion, im besonderen einer PIT Emulsion (Komponente c)), von zumindest einer Öl- oder Wachskomponente, ausgewählt aus der Gruppe, bestehend aus pflanzlichen, tierischen und mineralischen Ölen und Wachsen (Komponente e)), und von zumindest einer Silikonöl- und/oder Silikonwachskomponente (Komponente f)), um ein lipophiles Vorgemisch herzustellen, im besonderen durch Homogenisieren,
b1) Vermischen von Wasser (Komponente a)) und von zumindest einem grenzflächenaktiven Stoff (Komponente b)), um ein wässriges System auszubilden, und
c1) Vermischen des unter a) erhaltenen lipophilen Vorgemisches und des unter b) erhaltenen wässrigen Systems, um eine stabile kosmetische Zusammensetzung herzustellen, im besonderen durch das Hinzufügen des wässrigen Systems zum lipophilen Vorgemisch; oder
a2) Vermischen von zumindest einer Emulsion, im besonderen einer PIT Emulsion (Komponente c)), von zumindest einer Öl- oder Wachskomponente, ausgewählt aus der Gruppe, bestehend aus pflanzlichen, tierischen und mineralischen Ölen und Wachsen (Komponente e)), und von zumindest einer Silikonöl- und/oder Silikonwachskomponente (Komponente f)), um ein lipophiles Vorgemisch herzustellen, im besonderen durch Homogenisieren, und
b2) Vermischen des unter a) erhaltenen lipophilen Vorgemisches mit Wasser (Komponente a)) und zumindest einem grenzflächenaktiven Stoff (Komponente b)), um eine stabile kosmetische Zusammensetzung herzustellen, im besonderen durch das Hinzufügen von Wasser und einem grenzflächenaktiven Stoff zum lipophilen Vorgemisch.

28. Verfahren nach Anspruch 27, welches ferner folgenden Schritt umfasst:
Hinzufügen von zumindest einem chelatisierenden Mittel (Komponente g)), von zumindest einem Feuchtemittel (Komponente i)) und/oder von zumindest einem weiteren hydrophilen Bestandteil beim Schritt b1) oder b2) und/oder Hinzufügen von zumindest einem Konservierungsmittel (Komponente h)), von Parfüm und/oder von zumindest einem weiteren lipophilen Bestandteil beim Schritt a1) oder a2).

29. Verfahren nach Anspruch 27 oder 28, ferner umfassend:
den Schritt der Regelung des pH-Wertes der beim Schritt c1) oder b2) erhaltenen kosmetischen Zusammensetzung auf einen Bereich von 5,5 bis 7,5 durch das Hinzufügen eines pH-Reglers (f).

30. Verfahren nach einem der Ansprüche 27 bis 29, **dadurch gekennzeichnet, dass** zumindest das Vermischen beim Schritt a1) oder a2) durch Homogenisieren erzielt wird.

## Revendications

1. Composition cosmétique pour le démaquillage obtenue en mélangeant au moins les ingrédients suivants :
a) de l'eau
b) au moins un tensioactif,
c) au moins une émulsion,
d) au moins un composant huileux ou cireux choisi dans le groupe constitué par des huiles et cires végétales, animales et minérales, et
e) au moins un composant huileux et/ou cireux à base de silicone.

2. Composition cosmétique selon la revendication 1, comprenant en outre au moins un régulateur de pH (f).

3. Composition cosmétique selon la revendication 1 ou 2, comprenant en outre :
g) au moins un chélateur,
h) au moins un conservateur, et/ou
i) au moins un agent hydratant.

4. Composition cosmétique selon la revendication 3, dans laquelle le conservateur comprend un mélange comprenant du butylcarbamate d'iodopropynyle (IPBC) et de la DMDM hydantoïne, notamment dans une quantité allant d'environ 0,1 à 0,85 % en poids, en se basant sur le poids de la composition cosmétique, un mélange comprenant de l'IPBC, de l'acide benzoïque, de l'acide déhydroacétique et du phénoxyéthanol, et/ou un mélange comprenant du phénoxyéthanol, notamment dans une quantité d'environ 0,1 à 1,0 % en poids, en se basant sur le poids de la composition cosmétique, de l'IPBC, notamment dans une quantité d'environ 0,001 à 0,05 % en poids en se basant sur le poids de la composition cosmétique et de la DMDM hydantoïne, notamment dans une quantité d'environ 0,01 à 0,6 % en poids, en se basant sur le poids de la composition cosmétique.

5. Composition cosmétique selon l'une des revendications précédentes, dans laquelle l'émulsion c) comprend une microémulsion, une nanoémulsion ou une émulsion du type huile-dans-eau préparée par la technique d'inversion de phase (PIT), chacune comprenant au moins une phase huileuse.

6. Composition cosmétique selon l'une des revendications précédentes, dans laquelle l'émulsion c) comprend au moins un monoglycéride, un diglycéride et/ou un triglycéride d'acide gras et/ou au moins un éther polyalcoxylé d'un alcool gras en tant qu'émulsifiant(s) non ionique (s), notamment un alcool en C₁₆-C₂₂ polyéthoxylé.

7. Composition cosmétique selon l'une des revendications précédentes, dans laquelle l'émulsion c) comprend au moins un ester d'acide gras d'un alcool gras et/ou au moins un alcool gras, ou dans laquelle l'émulsion c) comprend au moins un ester d'un alcool gras, au moins un monoglycéride ou un diglycéride ou un mélange de ceux-ci, ou dans laquelle l'émulsion c) comprend au moins un alcool gras et au moins un alcool gras polyéthoxylé.

8. Composition cosmétique selon l'une des revendications précédentes, dans laquelle la phase huileuse de l'émulsion c) comprend au moins un ester d'un alcool gras, notamment l'isononanoate de cétéaryle.

9. Composition cosmétique selon l'une des revendications précédentes, dans laquelle l'émulsion c) comprend en outre au moins un éther de dialkyle(ène).

10. Composition cosmétique selon l'une des revendications précédentes, dans laquelle l'émulsion c) comprend au moins un composant huileux ou cireux choisi dans le groupe constitué par des huiles et des cires végétales, animales et minérales, notamment un composant huileux et/ou cireux à base de paraffine et/ou au moins un composant huileux et/ou cireux à base de silicone.

11. Composition cosmétique selon l'une des revendications précédentes, dans laquelle l'émulsion c) comprend en outre au moins un tensioactif, au moins un agent hydratant et/ou au moins un conservateur.

12. Composition cosmétique selon l'une des revendications précédentes, comprenant en outre au moins un agent antimicrobien, au moins un épaississant, au moins un parfum, au moins un agent adoucissant pour la peau, au moins un agent matifiant, au moins un agent anti-âge, au moins un agent anti-acnéique, au moins un agent protecteur anti-UV, au moins un humectant ou des mélanges de ceux-ci.

13. Composition cosmétique selon l'une des revendications précédentes, dans laquelle l'huile de silicone comprend le diméthicone, les diméthicones linéaires substitués, les cyclométhicones et des mélanges de ceux-ci, notamment le cyclopentasiloxane.

14. Composition cosmétique selon l'une des revendications précédentes, dans laquelle l'huile ou la cire minérale est une huile ou une cire de paraffine, notamment une huile ou une cire d'isoparaffine.

15. Composition cosmétique selon l'une des revendications précédentes, dans laquelle le composant à base d'huile de paraffine comprend, au moins, notamment un hydrocarbure en C₅-C₁₈ à substitution iso, en particulier l'isohexadécane.

16. Composition cosmétique selon l'une des revendications précédentes, dans laquelle les ingrédients sont mélangés selon les quantités suivantes :
a) 15 à 93,99 % en poids d'eau,
b) 0,01 à 10 % en poids de tensioactif,
c) 1 à 25 % en poids d'émulsion,
d) + e) 5 à 50 % en poids d'un mélange d'un composant huileux et/ou cireux à base de paraffine et d'un composant huileux et/ou cireux à base de silicone.

17. Composition cosmétique selon l'une des revendications précédentes, dans laquelle le rapport p/p des composants d) et e) se situe dans la plage de 100/1 à 1/100, de préférence dans la plage de 10/1 à 1/10.

18. Applicateur pour appliquer un soin cosmétique et/ou un soin dermatologique et/ou un produit nettoyant comprenant au moins une composition cosmétique selon l'une des revendications précédentes.

19. Applicateur selon la revendication 18 comprenant au moins un voile absorbant non tissé comprenant 10 à 100 % en poids de fibres de lyocell et 0 à 90 % en poids d'au moins une autre fibre naturelle, naturelle et/ou synthétique fabriquée, notamment une fibre polyester, dans lequel la fibre de lyocell a un titre de fibre dans la plage de 0,5 à 3,5 dtex, dans lequel le voile absorbant a un poids de base dans la plage de 20 à 500 g/m² et une résistance à la traction dans la plage de 5 à 1000 N/5 cm dans le sens machine et dans le sens travers.

20. Applicateur selon la revendication 18 ou 19, dans lequel le voile absorbant comprend 30 à 90 % en poids, notamment 40 à 80 % en poids de fibres de lyocell et 10 à 70 % en poids, notamment 20 à 60 % en poids d'autres fibres naturelles, naturelles et/ou synthétiques fabriquées, notamment des fibres synthétiques.

21. Applicateur selon l'une des revendications 18 à 20, dans lequel le voile absorbant comprend 10 à 50 % en poids, notamment 10 à 40 % en poids de fibres de lyocell, 10 à 40 % en poids de fibres naturelles, de préférence des fibres fabriquées à partir du coton, et 10 à 80 % en poids, notamment 20 à 80 % en poids de fibres naturelles et/ou synthétiques fabriquées, notamment une fibre polyester, dans lequel au moins un segment de la fibre naturelle, naturelle et/ou synthétique fabriquée comprend notamment au moins une surface hydrophile, en particulier permanente.

22. Applicateur selon l'une des revendications 18 à 21, dans lequel le voile absorbant a été fabriqué en utilisant le cardage qui est la technique de formation de la bande et l'aiguilletage qui est la technique de liaison de la bande et dans lequel les fibres cardées sont croisées avant l'aiguilletage.

23. Applicateur selon l'une des revendications 18 à 22, dans lequel les fibres du voile absorbant présentent un titre de fibre inférieur à 2,5 dtex, de préférence inférieur à 2,0 dtex et de manière encore préférée, inférieur ou égal à 1,3 dtex.

24. Applicateur selon l'une des revendications 18 à 23, dans lequel le voile absorbant a une épaisseur dans la plage de 0,4 à 5 mm, de préférence de 1,5 à 3 mm et de manière encore préférée, de 1,8 à 2,8 mm.

25. Applicateur selon l'une des revendications 18 à 24, dans lequel le moment de flexion du produit absorbant, notamment du voile absorbant, est inférieur ou égal à 0,20, de préférence inférieur ou égal à 0,17 et de manière encore préférée inférieur ou égal à 0,15 g x cm selon le test de Kawabata, notamment dans le sens machine et dans le sens travers.

26. Ensemble d'applicateurs comprenant deux, trois applicateurs ou plus selon l'une des revendications 18 à 25 qui sont au moins partiellement, directement ou indirectement, imposés les uns sur les autres, notamment agencés en au moins un empilement ou sur un rouleau.

27. Procédé pour la préparation de la composition cosmétique selon l'une des revendications 1 à 18 comprenant les étapes consistant à :
a1) mélanger au moins une émulsion, notamment une émulsion PIT (composant c)), au moins un composant huileux ou cireux choisi dans le groupe constitué par des huiles et des cires végétales, animales et minérales (composant e)) et au moins un composant huileux à base de silicone et/ou un composant cireux à base de silicone (composant f)) pour former un prémélange lipophile, notamment par homogénéisation,
b1) mélanger de l'eau (composant a) et au moins un tensioactif (composant b)) pour former un système aqueux, et
c1) mélanger le prémélange lipophile obtenu au point a) et le système aqueux obtenu au point b) pour former une composition cosmétique stable, notamment en ajoutant le système aqueux au pré mélange lipophile ; ou
a2) mélanger au moins une émulsion, notamment une émulsion PIT (composant c)), au moins un composant huileux ou cireux choisi dans le groupe constitué par des huiles et des cires végétales, animales et minérales (composant e)) et au moins un composant huileux à base de silicone et/ou un composant cireux à base de silicone (composant f)) pour former un pré mélange lipophile, notamment par homogénéisation, et
b2) mélanger le pré mélange lipophile obtenu au point a) avec l'eau (composant a)) et au moins un tensioactif (composant b)) pour former une composition cosmétique stable, notamment en ajoutant de l'eau et un tensioactif au pré mélange lipophile.

28. Procédé selon la revendication 27, comprenant en outre l'étape consistant à ajouter au moins un chélateur (composant g)), au moins un agent hydratant (composant i)) et/ou au moins un autre ingrédient hydrophile à l'étape b1) ou b2) et/ou à ajouter au moins un conservateur (composant h)), un parfum et/ou au moins un autre ingrédient lipophile à l'étape a1) ou a2).

29. Procédé selon la revendication 27 ou 28, comprenant en outre l'étape consistant à régler le pH de la composition cosmétique obtenue à l'étape c1) ou b2) dans une plage de 5,5 à 7,5 en ajoutant un régulateur de pH (f).

30. Procédé selon l'une des revendications 27 à 29, dans lequel on effectue au moins le mélange à l'étape a1) ou a2) en utilisant une homogénéisation.
